(19) Europäisches Patentamt
European Patent Office
Office européen des brevets

(11) EP 3 231 425 A1

(12) # EUROPEAN PATENT APPLICATION
published in accordance with Art. 153(4) EPC

(43) Date of publication:
**18.10.2017 Bulletin 2017/42**

(21) Application number: **15867918.3**

(22) Date of filing: **08.12.2015**

(51) Int Cl.:
***A61K 31/496*** *(2006.01)* ***A61K 31/454*** *(2006.01)*
***A61K 31/497*** *(2006.01)* ***A61P 35/00*** *(2006.01)*
***A61P 43/00*** *(2006.01)* ***C07D 401/14*** *(2006.01)*

(86) International application number:
**PCT/JP2015/084341**

(87) International publication number:
**WO 2016/093203 (16.06.2016 Gazette 2016/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **09.12.2014 JP 2014249350**

(71) Applicant: **Astellas Pharma Inc.**
**Chuo-ku**
**Tokyo 103-8411 (JP)**

(72) Inventors:
- **NAGASHIMA, Takeyuki**
  **Tokyo 103-8411 (JP)**
- **TSUJIMOTO, Susumu**
  **Tokyo 103-8411 (JP)**

(74) Representative: **Hoffmann Eitle**
**Patent- und Rechtsanwälte PartmbB**
**Arabellastraße 30**
**81925 München (DE)**

(54) **PHARMACEUTICAL COMPOSITION HAVING BICYCLIC NITROGEN-CONTAINING AROMATIC HETEROCYCLIC AMIDE COMPOUND AS ACTIVE COMPONENT**

(57) To provide a pharmaceutical composition for treating multiple myeloma. [Solution] After investigating compounds having a mitochondrial Complex I inhibitory effect, the present inventor completed the present invention by confirming that the bicyclic nitrogen-containing aromatic heterocyclic amide compounds of the present invention have a mitochondrial Complex I inhibitory effect, and that these compounds have a growth inhibition effect on multiple myeloma.

EP 3 231 425 A1

## Description

### Technical Field

The present invention relates to a pharmaceutical composition for treating multiple myeloma, comprising a bicyclic nitrogen-containing aromatic heterocyclic amide compound or a pharmaceutically acceptable salt thereof as an active ingredient. In addition, the invention is related to the combination use of a bicyclic nitrogen-containing aromatic heterocyclic amide compound or a pharmaceutically acceptable salt thereof and an agent for treating multiple myeloma, and to a combination treatment, which are for preventing or treating multiple myeloma.

### Background Art

Multiple myeloma is a tumor of plasma cells differentiated from B lymphocytes and is an incurable disease in which monoclonal immunoglobulin is produced by the plasma cells and various clinical symptoms such as hematopoietic disorders including anemia as a major disorder, frequent infections, kidney problems, osteolytic changes, and the like occur. It has been reported that multiple myeloma accounts for approximately 1% of all cancers and 10% or more of all hematopoietic malignancies (SEER Cancer Statistics Review, 2008-2012). In addition, the number of patients thereof has been increasing steadily in accordance with the increase in the elderly.

Recently, the introduction of new agents of immunomodulatory drugs such as lenalidomide and pomalidomide; proteasome inhibitors such as bortezomib and carfilzomib; and the like, and the combination use of these agents have improved a 5-year survival rate up to approximately 50% in the statistics in 2006 to 2010, whereas the rate was up to approximately 30% in the statistics in 2001 to 2005 (Leukemia, 2014, 28, 1122-1128). However, an average survival period of the patients for whom these agents are not effective is still short, and development of new agents exhibiting an effect on the patients with multiple myeloma that relapses and is resistant to treatments is strongly required. As administration forms, because many of the patients are the elderly and administration is performed over a long period of time in many cases, an oral agent that is less burden on the patients is required.

It has been known that in multiple myeloma, the phosphatidylinositol 3-kinase (PI3K)/Akt/mammalian target of rapamycin (mTOR) pathway is activated and is closely related to a disease state thereof (Clin. Cancer Res. 2007, 13: 3771-3775). For example, it has been reported that IL-6 stimulates the proliferation of myeloma cells via the PI3K/Akt/mTOR pathway (Oncogene, 2001, 20: 5991-6000). In addition, it has also been reported that IGF-I, known as a proliferative factor for myeloma cells, stimulates the PI3K/Akt/mTOR pathway (J.Immunol. 2004, 173: 4953-4959), and an inhibitor for IGF-I inhibits the proliferation of myeloma cells (Clin. Cancer Res. 2011, 17: 4693-4704).

It has been reported recently that metformin, known as the primary drug of choice of an agent for treating type II diabetes, activates adenosine monophosphate (AMP)-activated protein kinase (AMPK), and therefore inhibits the proliferation of breast cancer and lung cancer (Cancer Res. 2006, 66: 10269-10273, Expert Opin. Investig. Drugs. 2013, 22: 1401-1409). AMPK is a highly-preserved serine/threonine kinase, controls energy metabolism in various cells, and monitors changes in the AMP/ATP ratio in the cells and responds to it (Annu. Rev. Biochem. 1998, 67: 821-855). The activation of AMPK by metformin is known to be based on the effect of inhibiting mitochondrial Complex I (Diabetes Metab. 2003,29 (4 Pt 2): 6S88-94). Mitochondrial Complex I is a NADH dehydrogenase located in the mitochondrial inner membrane and is known as the "entry enzyme" for oxidative phosphorylation in the mitochondria. The inhibition of mitochondrial Complex I leads to the inhibition of oxidative phosphorylation that is ATP production reaction in the electron transport system of the mitochondria. As ATP levels in the cells decrease, AMP/ATP ratio increases, and AMP binding to AMPK allosterically activates AMPK. The activated AMPK inhibits mTOR signaling via phosphorylation of tuberous sclerosis complex 2 (TSC2) that is downstream of the PI3K/Akt pathway (Genes Cells. 2003, 8: 65-79). This is considered to be one of the reasons why metformin inhibits the proliferation of cancer cells (Cancer Res. 2007, 67: 10804-10812).

As a compound having the effect of inhibiting mitochondrial Complex I, regardless of whether they are natural or unnatural, many types of compounds such as rotenone, pyridaben, bullatacin, piericidin A, capsaicin, fenazaquin, and the like are known. In addition, for example, it has been reported that a compound of Formula (A) below has the effect of inhibiting mitochondrial Complex I and inhibits the proliferation of various types of cancer cells (Patent Document 1).

[Chem. 1]

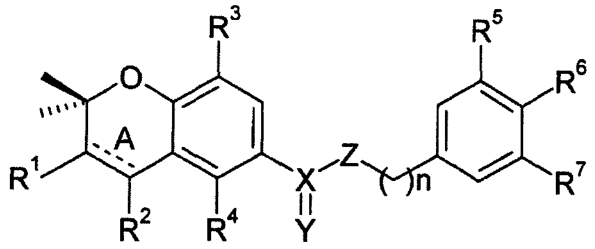

(A)

(refer to the corresponding publication for the meaning of symbols in the formula)

In addition, as a compound having the effect of activating AMPK, it has been reported that for example, a compound of Formulas (B) and (C) below has the effect of activating AMPK, and is useful for treating a metabolic disorder such as type II diabetes, atherosclerosis, cardiovascular disease, and the like (refer to Patent Document 2 and Patent Document 3, respectively). However, in the documents, there is no specific description that teaches the usefulness for treating cancer and the like.

[Chem. 2]

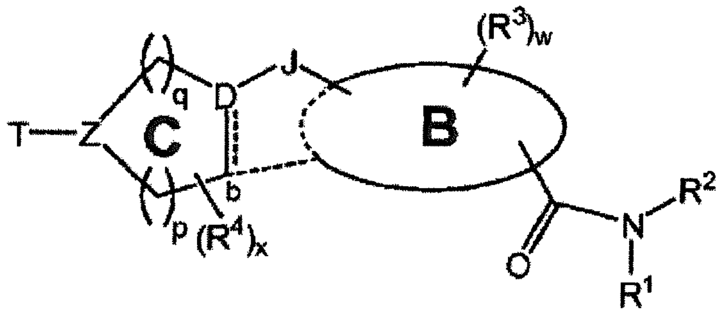

(B)

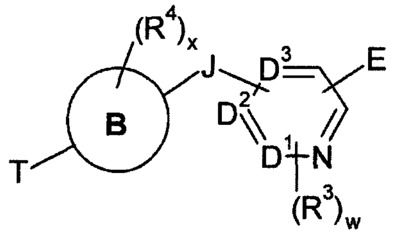

(C)

(refer to the corresponding publication for the meaning of symbols in the formulas)

Related Art Document

Patent Document

Patent Document 1: International Publication No. 02/20008
Patent Document 2: International Publication No. 2009/132136
Patent Document 3: International Publication No. 2012/016217

Summary of Invention

Problems to Be Solved by the Invention

A pharmaceutical composition for treating multiple myeloma, particularly a pharmaceutical composition for

treating multiple myeloma in which the PI3K/Akt/mTOR pathway is enhanced is provided.

Means for Solving the Problems

As a result of intensive examination for creating a pharmaceutical composition for treating multiple myeloma, the inventors of the present invention have found that a bicyclic nitrogen-containing aromatic heterocyclic amide compound of the present invention or a pharmaceutically acceptable salt thereof exhibits an excellent effect of inhibiting mitochondrial Complex I (WO 2014/199933 published by the applicant of the present application after the patent application that is the basis of the priority of the present application), that this compound exhibits an effect of inhibiting growth of multiple myeloma, and that a remarkable enhancement of an anti-tumor effect is achieved by using lenalidomide in combination, and therefore have completed the present invention.

That is, the present invention is related to a pharmaceutical composition for treating multiple myeloma, comprising a compound selected from (5-{1-[(6-methoxypyridin-3-yl)methyl]piperidin-4-yl}-1H-benzimidazol-2-yl){4-[4-(trifluoromethyl)benzyl]piperazin-1-yl}methanone (hereinafter will be referred to as "Compound A"), (5-{1-[(5-methoxypyrazin-2-yl)methyl]piperidin-4-yl}-1H-benzimidazol-2-yl){4-[4-(trifluoromethyl)benzyl]piperazin-1-yl}methanone (hereinafter will be referred to as "Compound B"), 4-({4-[(6-{1-[(5-ethoxypyrazin-2-yl)methyl]piperidin-4-yl}-1-methyl-1H-indol-2-yl)carbonyl]piperazin-1-yl}methyl)benzonitrile (hereinafter will be referred to as "Compound C"), and 4-({4-[(5-{1-[(5-methoxypyrazin-2-yl)methyl]piperidin-4-yl}-1-methyl-1H-indol-2-yl)carbonyl]piperazin-1-yl}methyl)benzonitrile (hereinafter will be referred to as "Compound D"), or a pharmaceutically acceptable salt thereof as an active ingredient, and in another embodiment, a pharmaceutical composition for treating multiple myeloma in which the PI3K/Akt/mTOR pathway is enhanced.

In addition, the present invention includes an agent for treating multiple myeloma, comprising a compound selected from Compound A, Compound B, Compound C, and Compound D or a pharmaceutically acceptable salt thereof, and in another embodiment, an agent for treating multiple myeloma in which the PI3K/Akt/mTOR pathway is enhanced.

In addition, the present invention relates to the use of a compound selected from Compound A, Compound B, Compound C, and Compound D or a pharmaceutically acceptable salt thereof for the manufacture of a pharmaceutical composition for treating multiple myeloma, and in another embodiment, for the manufacture of a pharmaceutical composition for treating multiple myeloma in which the PI3K/Akt/mTOR pathway is enhanced; the use of a compound selected from Compound A, Compound B, Compound C, and Compound D or a pharmaceutically acceptable salt thereof for treating multiple myeloma, and in another embodiment, for treating multiple myeloma in which the PI3K/Akt/mTOR pathway is enhanced; a compound selected from Compound A, Compound B, Compound C, and Compound D or a pharmaceutically acceptable salt thereof for treating multiple myeloma, and in another embodiment, for treating multiple myeloma in which the PI3K/Akt/mTOR pathway is enhanced; and a method for treating multiple myeloma, and in another embodiment, a method for treating multiple myeloma in which the PI3K/Akt/mTOR pathway is enhanced by administering an effective dose of a compound selected from Compound A, Compound B, Compound C, and Compound D or a pharmaceutically acceptable salt thereof to a subject. The term "subject" refers to humans or any other animals in need of the treatment, and in another embodiment, refers to humans in need of the treatment.

Furthermore, the present invention relates to the use of a compound selected from Compound A, Compound B, Compound C, and Compound D or a pharmaceutically acceptable salt thereof for the manufacture of a pharmaceutical composition for preventing or treating multiple myeloma, the compound or the salt being characterized by being used in combination with an agent for treating multiple myeloma, and in another embodiment, for the manufacture of a pharmaceutical composition for preventing or treating multiple myeloma in which the PI3K/Akt/mTOR pathway is enhanced, the compound or the salt being characterized by being used in combination with an agent for treating multiple myeloma; the use of a compound selected from Compound A, Compound B, Compound C, and Compound D or a pharmaceutically acceptable salt thereof for preventing or treating multiple myeloma, the compound or the salt being characterized by being used in combination with an agent for treating multiple myeloma, and in another embodiment, for preventing or treating multiple myeloma in which the PI3K/Akt/mTOR pathway is enhanced, the compound or the salt being characterized by being used in combination with an agent for treating multiple myeloma; and a pharmaceutical composition for preventing or treating multiple myeloma, comprising a compound selected from Compound A, Compound B, Compound C, and Compound D or a pharmaceutically acceptable salt thereof, which is characterized by being used in combination with an agent for treating multiple myeloma, as an active ingredient, and in another embodiment, a pharmaceutical composition for preventing or treating multiple myeloma in which the PI3K/Akt/mTOR pathway is enhanced.

Effects of the Invention

A compound selected from Compound A, Compound B, Compound C, and Compound D or a pharmaceutically acceptable salt thereof, which are active ingredients of a pharmaceutical composition of the present invention, has the

effect of inhibiting mitochondrial Complex I, exhibits an effect of inhibiting growth of multiple myeloma, and can be used as an active ingredient of a pharmaceutical composition for treating multiple myeloma, and in another embodiment, of a pharmaceutical composition for treating multiple myeloma in which the PI3K/Akt/mTOR pathway is enhanced.

Embodiments for Carrying Out the Invention

Hereinafter, the present invention will be described in detail.

An agent for treating multiple myeloma includes melphalan, prednisolone, dexamethasone, thalidomide, lenalidomide, pomalidomide, bortezomib, carfilzomib, and the like, and includes lenalidomide in another embodiment.

Lenalidomide is an agent for treating multiple myeloma. Lenalidomide can be commercially purchased and used or can be easily obtained by an obvious method or a modification method thereof. In addition, lenalidomide is already being used clinically, and administration route, administration cycle, and dose thereof are obvious for those skilled in the art.

In the present specification, "a pharmaceutically acceptable salt of a compound selected from Compound A, Compound B, Compound C, and Compound D" means an acid addition salt of Compound A, Compound B, Compound C, or Compound D. Specific examples of the acid addition salt include the salts with inorganic acids such as hydrochloric acid, hydrobromic acid, hydroiodic acid, sulfuric acid, nitric acid, phosphoric acid and the like, and organic acids such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditoluoyltartaric acid, citric acid, methanesulfonic acid (mesylic acid), ethanesulfonic acid, benzenesulfonic acid, p-toluenesulfonic acid (tosylic acid), aspartic acid, glutamic acid, and the like.

Examples of "a compound selected from Compound A, Compound B, Compound C, and Compound D" include various solvates of Compound A, Compound B, Compound C, or Compound D, and specifically, include a hydrate or an ethanol solvate. Furthermore, "a pharmaceutically acceptable salt" includes an acid addition salt of these solvates.

In addition, examples of "a compound selected from Compound A, Compound B, Compound C, and Compound D or a pharmaceutically acceptable salt thereof" include freebase in which a salt is not formed, that is, Compound A, Compound B, Compound C, or Compound D in a certain embodiment, Compound A in another embodiment, Compound B in still another embodiment, and Compound C in still further another embodiment, and Compound D in still further another embodiment. Furthermore, a tosylate salt of Compound A, Compound B, Compound C, or Compound D is included in another embodiment, a ditosylate salt of Compound A in still another embodiment, a ditosylate salt of Compound B in still further another embodiment, a ditosylate salt of Compound C in still further another embodiment, and a ditosylate salt of Compound D in still further another embodiment.

In addition, hereinafter, "a compound selected from Compound A, Compound B, Compound C, and Compound D or a pharmaceutically acceptable salt thereof" will be referred to as "a bicyclic nitrogen-containing aromatic heterocyclic amide compound or a pharmaceutically acceptable salt thereof".

The embodiments of the present invention are presented as below.

(1-1) A pharmaceutical composition for treating multiple myeloma, comprising Compound A or a pharmaceutically acceptable salt thereof as an active ingredient. In another embodiment, a pharmaceutical composition for treating multiple myeloma in which the PI3K/Akt/mTOR pathway is enhanced, comprising Compound A or a pharmaceutically acceptable salt thereof as an active ingredient. In still another embodiment, a pharmaceutical composition for treating multiple myeloma, comprising a ditosylate salt of Compound A as an active ingredient. In still further another embodiment, a pharmaceutical composition for treating multiple myeloma in which the PI3K/Akt/mTOR pathway is enhanced, comprising a ditosylate salt of Compound A as an active ingredient.

(1-2) The use of Compound A or a pharmaceutically acceptable salt thereof for the manufacture of a pharmaceutical composition for treating multiple myeloma. In another embodiment, the use of Compound A or a pharmaceutically acceptable salt thereof for the manufacture of a pharmaceutical composition for treating multiple myeloma in which the PI3K/Akt/mTOR pathway is enhanced. In still another embodiment, the use of a ditosylate salt of Compound A for the manufacture of a pharmaceutical composition for treating multiple myeloma. In still further another embodiment, the use of a ditosylate salt of Compound A for the manufacture of a pharmaceutical composition for treating multiple myeloma in which the PI3K/AktImTOR pathway is enhanced.

(1-3) The use of Compound A or a pharmaceutically acceptable salt thereof for treating multiple myeloma. In another embodiment, the use of Compound A or a pharmaceutically acceptable salt thereof for treating multiple myeloma in which the PI3K/Akt/mTOR pathway is enhanced. In still another embodiment, the use of a ditosylate salt of Compound A for treating multiple myeloma. In still further another embodiment, the use of a ditosylate salt of Compound A for treating multiple myeloma in which the PI3K/Akt/mTOR pathway is enhanced.

(1-4) Compound A or a pharmaceutically acceptable salt thereof for treating multiple myeloma. In another embodiment, Compound A or a pharmaceutically acceptable salt thereof for treating multiple myeloma in which the

PI3K/Akt/mTOR pathway is enhanced. In still another embodiment, a ditosylate salt of Compound A for treating multiple myeloma. In still further another embodiment, a ditosylate salt of Compound A for treating multiple myeloma in which the PI3K/Akt/mTOR pathway is enhanced.

(1-5) A method for treating multiple myeloma by administering an effective dose of Compound A or a pharmaceutically acceptable salt thereof to a subject. In another embodiment, a method for treating multiple myeloma in which the PI3K/Akt/mTOR pathway is enhanced by administering an effective dose of Compound A or a pharmaceutically acceptable salt thereof to a subject. In still another embodiment, a method for treating multiple myeloma by administering an effective dose of a ditosylate salt of Compound A to a subject. In still further another embodiment, a method for treating multiple myeloma in which the PI3K/Akt/mTOR pathway is enhanced by administering an effective dose of a ditosylate salt of Compound A to a subject.

(2-1) A pharmaceutical composition for treating multiple myeloma, comprising Compound B or a pharmaceutically acceptable salt thereof as an active ingredient. In another embodiment, a pharmaceutical composition for treating multiple myeloma in which the PI3K/Akt/mTOR pathway is enhanced, comprising Compound B or a pharmaceutically acceptable salt thereof as an active ingredient. In still another embodiment, a pharmaceutical composition for treating multiple myeloma, comprising a ditosylate salt of Compound B as an active ingredient. In still further another embodiment, a pharmaceutical composition for treating multiple myeloma in which the PI3K/Akt/mTOR pathway is enhanced, comprising a ditosylate salt of Compound B as an active ingredient.

(2-2) The use of Compound B or a pharmaceutically acceptable salt thereof for the manufacture of a pharmaceutical composition for treating multiple myeloma. In another embodiment, the use of Compound B or a pharmaceutically acceptable salt thereof for the manufacture of a pharmaceutical composition for treating multiple myeloma in which the PI3K/Akt/mTOR pathway is enhanced. In still another embodiment, the use of a ditosylate salt of Compound B for the manufacture of a pharmaceutical composition for treating multiple myeloma. In still further another embodiment, the use of a ditosylate salt of Compound B for the manufacture of a pharmaceutical composition for treating multiple myeloma in which the PI3K/Akt/mTOR pathway is enhanced.

(2-3) The use of Compound B or a pharmaceutically acceptable salt thereof for treating multiple myeloma. In another embodiment, the use of Compound B or a pharmaceutically acceptable salt thereof for treating multiple myeloma in which the PI3K/AktImTOR pathway is enhanced. In still another embodiment, the use of a ditosylate salt of Compound B for treating multiple myeloma. In still further another embodiment, the use of a ditosylate salt of Compound B for treating multiple myeloma in which the PI3K/Akt/mTOR pathway is enhanced.

(2-4) Compound B or a pharmaceutically acceptable salt thereof for treating multiple myeloma. In another embodiment, Compound B or a pharmaceutically acceptable salt thereof for treating multiple myeloma in which the PI3K/Akt/mTOR pathway is enhanced. In still another embodiment, a ditosylate salt of Compound B for treating multiple myeloma. In still further another embodiment, a ditosylate salt of Compound B for treating multiple myeloma in which the PI3K/Akt/mTOR pathway is enhanced.

(2-5) A method for treating multiple myeloma by administering an effective dose of Compound B or a pharmaceutically acceptable salt thereof to a subject. In another embodiment, a method for treating multiple myeloma in which the PI3K/Akt/mTOR pathway is enhanced by administering an effective dose of Compound B or a pharmaceutically acceptable salt thereof to a subject. In still another embodiment, a method for treating multiple myeloma by administering an effective dose of a ditosylate salt of Compound B to a subject. In still further another embodiment, a method for treating multiple myeloma in which the PI3K/Akt/mTOR pathway is enhanced by administering an effective dose of a ditosylate salt of Compound B to a subject.

(3-1) A pharmaceutical composition for treating multiple myeloma, comprising Compound C or a pharmaceutically acceptable salt thereof as an active ingredient. In another embodiment, a pharmaceutical composition for treating multiple myeloma in which the PI3K/Akt/mTOR pathway is enhanced, comprising Compound C or a pharmaceutically acceptable salt thereof as an active ingredient. In still another embodiment, a pharmaceutical composition for treating multiple myeloma, comprising a ditosylate salt of Compound C as an active ingredient. In still further another embodiment, a pharmaceutical composition for treating multiple myeloma in which the PI3K/Akt/mTOR pathway is enhanced, comprising a ditosylate salt of Compound C as an active ingredient.

(3-2) The use of Compound C or a pharmaceutically acceptable salt thereof for the manufacture of a pharmaceutical composition for treating multiple myeloma. In another embodiment, the use of Compound C or a pharmaceutically acceptable salt thereof for the manufacture of a pharmaceutical composition for treating multiple myeloma in which the PI3K/Akt/mTOR pathway is enhanced. In still another embodiment, the use of a ditosylate salt of Compound C for the manufacture of a pharmaceutical composition for treating multiple myeloma. In still further another embodiment, the use of a ditosylate salt of Compound C for the manufacture of a pharmaceutical composition for treating multiple myeloma in which the PI3K/Akt/mTOR pathway is enhanced.

(3-3) The use of Compound C or a pharmaceutically acceptable salt thereof for treating multiple myeloma. In another embodiment, the use of Compound C or a pharmaceutically acceptable salt thereof for treating multiple myeloma in which the PI3K/Akt/mTOR pathway is enhanced. In still another embodiment, the use of a ditosylate salt of

Compound C for treating multiple myeloma. In still further another embodiment, the use of a ditosylate salt of Compound C for treating multiple myeloma in which the PI3K/Akt/mTOR pathway is enhanced.

(3-4) Compound C or a pharmaceutically acceptable salt thereof for treating multiple myeloma. In another embodiment, Compound C or a pharmaceutically acceptable salt thereof for treating multiple myeloma in which the PI3K/Akt/mTOR pathway is enhanced. In still another embodiment, a ditosylate salt of Compound C for treating multiple myeloma. In still further another embodiment, a ditosylate salt of Compound C for treating multiple myeloma in which the PI3K/Akt/mTOR pathway is enhanced.

(3-5) A method for treating multiple myeloma by administering an effective dose of Compound C or a pharmaceutically acceptable salt thereof to a subject. In another embodiment, a method for treating multiple myeloma in which the PI3K/Akt/mTOR pathway is enhanced by administering an effective dose of Compound C or a pharmaceutically acceptable salt thereof to a subject. In still another embodiment, a method for treating multiple myeloma by administering an effective dose of a ditosylate salt of Compound C to a subject. In still further another embodiment, a method for treating multiple myeloma in which the PI3K/Akt/mTOR pathway is enhanced by administering an effective dose of a ditosylate salt of Compound C to a subject.

(4-1) A pharmaceutical composition for treating multiple myeloma, comprising Compound D or a pharmaceutically acceptable salt thereof as an active ingredient. In another embodiment, a pharmaceutical composition for treating multiple myeloma in which the PI3K/AktImTOR pathway is enhanced, comprising Compound D or a pharmaceutically acceptable salt thereof as an active ingredient. In still another embodiment, a pharmaceutical composition for treating multiple myeloma, comprising a ditosylate salt of Compound D as an active ingredient. In still further another embodiment, a pharmaceutical composition for treating multiple myeloma in which the PI3K/Akt/mTOR pathway is enhanced, comprising a ditosylate salt of Compound D as an active ingredient.

(4-2) The use of Compound D or a pharmaceutically acceptable salt thereof for the manufacture of a pharmaceutical composition for treating multiple myeloma. In another embodiment, the use of Compound D or a pharmaceutically acceptable salt thereof for the manufacture of a pharmaceutical composition for treating multiple myeloma in which the PI3K/Akt/mTOR pathway is enhanced. In still another embodiment, the use of a ditosylate salt of Compound D for the manufacture of a pharmaceutical composition for treating multiple myeloma. In still further another embodiment, the use of a ditosylate salt of Compound D for the manufacture of a pharmaceutical composition for treating multiple myeloma in which the PI3K/Akt/mTOR pathway is enhanced.

(4-3) The use of Compound D or a pharmaceutically acceptable salt thereof for treating multiple myeloma. In another embodiment, the use of Compound D or a pharmaceutically acceptable salt thereof for treating multiple myeloma in which the PI3K/Akt/mTOR pathway is enhanced. In still another embodiment, the use of a ditosylate salt of Compound D for treating multiple myeloma. In still further another embodiment, the use of a ditosylate salt of Compound D for treating multiple myeloma in which the PI3K/Akt/mTOR pathway is enhanced.

(4-4) Compound D or a pharmaceutically acceptable salt thereof for treating multiple myeloma. In another embodiment, Compound D or a pharmaceutically acceptable salt thereof for treating multiple myeloma in which the PI3K/Akt/mTOR pathway is enhanced. In still another embodiment, a ditosylate salt of Compound D for treating multiple myeloma. In still further another embodiment, a ditosylate salt of Compound D for treating multiple myeloma in which the PI3K/Akt/mTOR pathway is enhanced.

(4-5) A method for treating multiple myeloma by administering an effective dose of Compound D or a pharmaceutically acceptable salt thereof to a subject. In another embodiment, a method for treating multiple myeloma in which the PI3K/Akt/mTOR pathway is enhanced by administering an effective dose of Compound D or a pharmaceutically acceptable salt thereof to a subject. In still another embodiment, a method for treating multiple myeloma by administering an effective dose of a ditosylate salt of Compound D to a subject. In still further another embodiment, a method for treating multiple myeloma in which the PI3K/AktImTOR pathway is enhanced by administering an effective dose of a ditosylate salt of Compound D to a subject.

(5-1) A pharmaceutical composition for treating multiple myeloma, comprising a compound selected from Compound A, Compound B, Compound C, and Compound D or a pharmaceutically acceptable salt thereof, which is characterized by being used in combination with lenalidomide, as an active ingredient.

(5-2) A pharmaceutical composition for treating multiple myeloma described in (5-1), the composition being characterized by being administered simultaneously with administration of lenalidomide, successively, or over a time interval.

(5-3) The use of a compound selected from Compound A, Compound B, Compound C, and Compound D or a pharmaceutically acceptable salt thereof, which is characterized by being used in combination with lenalidomide, for the manufacture of a pharmaceutical composition for treating multiple myeloma.

(5-4) A compound selected from Compound A, Compound B, Compound C, and Compound D or a pharmaceutically acceptable salt thereof, which is characterized by being used in combination with lenalidomide, for treating multiple myeloma.

(5-5) The use of a compound selected from Compound A, Compound B, Compound C, and Compound D or a

pharmaceutically acceptable salt thereof, which is characterized by being used in combination with lenalidomide, for treating multiple myeloma.

(5-6) A pharmaceutical composition for treating multiple myeloma, comprising a compound selected from Compound A, Compound B, Compound C, and Compound D or a pharmaceutically acceptable salt thereof, and lenalidomide, as an active ingredient.

(5-7) A method for treating multiple myeloma which is characterized by administration of an effective treatment dose of lenalidomide, and an effective treatment dose of a compound selected from Compound A, Compound B, Compound C, and Compound D or a pharmaceutically acceptable salt thereof in combination, to a subject.

(5-8) A method for treating multiple myeloma described in (5-7) which is characterized by administration of the composition simultaneously with administration of lenalidomide, successively, or over a time interval, to a subject.

(5-9) A pharmaceutical composition for treating multiple myeloma which is for treating a subject subjected to a multiple myeloma treatment by lenalidomide, comprising a compound selected from Compound A, Compound B, Compound C, and Compound D or a pharmaceutically acceptable salt thereof as an active ingredient.

(5-10) A pharmaceutical composition described in (5-1), (5-2), (5-6), and (5-9) which is for treating multiple myeloma in which the PI3K/Akt/mTOR pathway is enhanced.

(5-11) An enhancer for an anti-multiple myeloma effect of lenalidomide, comprising a compound selected from Compound A, Compound B, Compound C, and Compound D or a pharmaceutically acceptable salt thereof as an active ingredient.

(5-12) A kit comprising a pharmaceutical composition comprising a compound selected from Compound A, Compound B, Compound C, and Compound D or a pharmaceutically acceptable salt thereof as an active ingredient, and a pharmaceutical composition comprising lenalidomide as an active ingredient in combination.

(5-13) A pharmaceutical product for treating multiple myeloma, comprising a package insert in which administration of a pharmaceutical composition comprising a compound selected from Compound A, Compound B, Compound C, and Compound D or a pharmaceutically acceptable salt thereof as an active ingredient, and a pharmaceutical composition comprising lenalidomide as an active ingredient in combination is described.

Pharmacological effects of a pharmaceutical composition of the present invention are confirmed by Test Examples below. In the Test Examples below, a ditosylate salt of Compound A (hereinafter will be referred to as Compound A1), a ditosylate salt of Compound B (hereinafter will be referred to as Compound B1), a ditosylate salt of Compound C (hereinafter will be referred to as Compound C1), and a ditosylate salt of Compound D (hereinafter will be referred to as "Compound D1) were used as a test compound. In each Test Example, the concentration of Compounds A1, B1, C1, and D1 is described in terms of the concentration of each freebase.

Test Example 1 Evaluation on Effect of Inhibiting Human Mitochondrial Complex I

Mitochondria were extracted from MDA-MB-453 tumor that is Human PIK3CA mutation-positive breast cancer, and the activation of inhibiting Complex I by Compounds A1, B1, C1, and D1 was evaluated.

PIK3CA mutation-positive breast cancer refers to breast cancer having mutations in PIK3CA, a gene name of p110$\alpha$ which is the catalytic subunit of PI3K, among mutations in phosphatidylinositol 3-kinase (PI3K) pathway genes.

In addition, MDA-MB-453 cells used in this Test Example and the next Test Example 2 were obtained from the American Type Culture Collection (hereinafter will be referred to as ATCC).

4-week-old male nude mice (Charles River Laboratories Japan, Inc.) were treated to bear the tumor of MDA-MB-453 cells derived from human PIK3CA mutation-positive breast cancer under their skin, and after MDA-MB-453 tumor reached a certain size, it was extracted. A solution for extraction of mitochondria (0.275 M Sucrose, 2.2 mM EDTA, 11 mM Tris/HCl, pH 7.5, Complete-EDTA-free (Roche Diagnostics)) was added thereto, by 9 times as much as the tumor weight, and then the tumor was crushed. The centrifugation was performed at 600 x g for 10 minutes at 4°C, the supernatant was obtained, and then the centrifugation was performed at 14,000 x g for 10 minutes at 4°C, and pellets were obtained. The pellets were suspended in 10 mM Tris/HCl pH 7.5 in the amount of 5 times as much as the weight of the extracted tumor, and a suspension of human mitochondria was obtained.

Next, 25 or 50 $\mu$l of the suspension of human mitochondria was added per 1 ml of a solution for measuring Complex I activity (25 mM potassium phosphate, pH 7.6, 0.35% Bovine Serum Albumin (BSA), 60 $\mu$M 2,6-dichlorophenol-indophenol, 70 $\mu$M decylubiquinone, 1 $\mu$M antimycin). After the solution was collected and added to a 96 or 384 well plate, a test compound was added to an arbitrary range from the final concentration of 10,000 nM to the final concentration of 0.3 nM. As a negative control, dimethylsulfoxide (DMSO) that is a solvent for the test compound was added to make a final concentration of 1%, and as a positive control, rotenone that is a Complex I inhibitor was added to make a final concentration of 1 $\mu$M. Furthermore, NADH was added to each well to make a final concentration of 0.2 or 0.5 mM, and the change in absorbance at a wavelength of 600 nm was measured by using the SpectraMax (Molecular Devices, LLC.) set to 37°C in advance. Signal values in a DMSO treatment were set as the top value, and signal values in a rotenone

1 μM treatment were set as the bottom value. Fluctuations in the signals were calculated within a range where the reaction was linear, and 50% inhibition values ($IC_{50}$) were calculated by a nonlinear regression analysis method using a Sigmoid Emax model. The result of Test Compounds A1, B1, C1, and D1 is shown in Table 1. Compounds A1 to D1 were produced by using a method described in Examples 1 to 4 described below (the same shall apply hereinafter).

[Table 1]

| Test Compound | $IC_{50}$ (nM) |
|---|---|
| A1 | 41 |
| B1 | 75 |
| C1 | 22 |
| D1 | 120 |

Test Example 2 Evaluation on Effect of Activating AMPK

Phosphorylation of 79th serine (Ser79) of Acetyl-CoA Carboxylase (ACC) which is a substrate of AMPK was measured using Cell ELISA, and thereby the effect of activating AMPK by Compounds A1, B1, C1, and D1 was evaluated.

36 μl each of MDA-MB-453 cells was seeded in Leibovitz's L-15 medium including 10% fetal bovine serum (Life Technologies Corporation) in a 384 well plate so that the cells became 15,000 cells per well, and the cells were cultured overnight at 37°C in the absence of $CO_2$. On the following day, Test Compounds A1, B1, C1, and D1, and DMSO which is a solvent for the test compounds as a negative control were diluted to a 10-fold concentration of a final concentration with a fresh medium, and 4 μl of the resultant product was added to each well (the test compounds had 10 steps in a final concentration from 10,000 nM to 0.3 nM, and DMSO had a final concentration of 0.1%). After that, the cells were cultured at 37°C for 2 hours in the absence of $CO_2$. 20 μl of a 40% glyoxal solution (Nacalai Tesque, INC.) was added to each well, and then the cells were left to stand at room temperature for 30 minutes to be fixed. Thereafter, the supernatant was removed by centrifuging the plate (at 800 rpm for 8 seconds by using the Ecospin of C.A.N. Hereinafter the centrifugation was performed under the same conditions unless otherwise specified), and 20 μl of 0.1 % Triton X-100-containing Phosphate-Buffered Saline (PBS) was added to each well, and then left to stand at room temperature for 10 minutes. The 0.1% Triton X-100-containing PBS was removed by centrifugation, and 20 μl of a blocking solution (Odyssey Blocking Buffer manufactured by LI-COR Biosciences, Inc.) was added to each well, and then left to stand at room temperature for 1 hour. The blocking solution was removed by centrifugation, and 10 μl of a blocking solution in which the amount of a phosphorylation antibody (manufactured by Cell Signaling Technology, Inc.) of ACC Ser79 as a primary antibody is 1/500 with respect to the undiluted solution, was added to each well, and then left to stand at 4°C overnight. On the following day, the reaction solution was removed by centrifuging the plate, and 25 μl of 0.05% Tween-20-containing Tris-Buffered Saline (TBS) (manufactured by Thermo Scientific; used in 1x by diluting 20xTBS Tween-20 with ion-exchange water) was added to each well, and then each well was washed by centrifugal removal. The washing was performed for a total of 3 times. After washing, 10 μl of a blocking solution containing IRDye (registered trademark) 800CW Goat anti-Rabbit IgG (manufactured by LI-COR Biosciences, Inc.) as a secondary antibody in the amount of 1/1000 with respect to the undiluted solution was added to each well, and then left to stand at room temperature for 1 hour. The reaction solution was removed by centrifuging the plate, and then each well was washed 3 times with 0.05% Tween-20-containing TBS in the same manner as after the primary antibody reaction. After the washing solution was removed, the plate was air-dried at room temperature for 3 hours or longer and signals were measured by Aerius (manufactured by LI-COR Biosciences, Inc.). Signal values in a DMSO treatment were set to a bottom value, and signal values when reaching a plateau were set to a top value, and 50% activation values ($EC_{50}$) were calculated by a nonlinear regression analysis method using a Sigmoid Emax model. The result of Test Compounds A1, B1, C1, and D1 is shown in Table 2.

[Table 2]

| Test Compound | $EC_{50}$ (nM) |
|---|---|
| A1 | 24 |
| B1 | 8.3 |
| C1 | 1.8 |
| D1 | 3.3 |

Test Example 3 Anti-Tumor Test on Mice Bearing Tumors of Human Myeloma Cell (1)

KMS-12-BM cells (purchased) or OPM-2 cells (purchased) derived from human myeloma were prepared at 5 x $10^7$ cells/mL of a mixed solution of PBS and Matrigel (registered trademark) in 1:1 ratio, and 0.1 mL was implanted to CAnN.Cg-Foxn1$^{nu}$/CrlCrlj mice (4-week-old male nude mice, Charles River Laboratories Japan, Inc.) under the skin on the back (5 x $10^6$ cells/head). Dividing into groups by the tumor volume was performed when tumor cells were engrafted and the average tumor volume exceeded approximately 300 $mm^3$ (5 mice for each group). The test compound dissolved in 6% cyclodextrin aqueous solution (Sigma-Aldrich Co.) was orally administered by the dosage of 8 mg/kg once a day for 21 days in the test using KMS-12-BM cells, and for 7 days in the test using OPM-2 cells, respectively. 6% Cyclodextrin aqueous solution was orally administered for a solvent group (control group) once a day.

RPMI 8226 cells (purchased) derived from human myeloma were prepared at 5 x $10^7$ cells/mL of a mixed solution of PBS and Matrigel (registered trademark) in 1:1 ratio, and 0.1 mL was implanted to CB17/Icr-Prkdc$^{scid}$/CrlCrlj mice (5-week-old male SCID mice, Charles River Laboratories Japan, Inc.) under the skin on the back (5 x $10^6$ cells/head). Dividing into groups by the tumor volume was performed when tumor cells were engrafted and the average tumor volume exceeded approximately 150 $mm^3$ (5 mice for each group). The test compound dissolved in 6% cyclodextrin aqueous solution was orally administered by the dosage of 8 mg/kg once a day for 21 days continuously. 6% Cyclodextrin aqueous solution was orally administered for a solvent group (control group) once a day.

The body weight and the tumor diameter were measured twice a week, and the anti-tumor effect and the effect on the body weight were examined. For calculation of the tumor volume, the formula below was used.

$$[\text{Tumor volume (mm}^3)] = [\text{major axis of tumor (mm)}] \times [\text{minor axis of tumor (mm)}]^2 \times 0.5$$

The inhibition rate of tumor growth and the rate of tumor regression were calculated from the average value of the tumor volume according to the formula below. The rate of tumor regression was calculated with respect to a group having an inhibition rate of tumor growth > 100%.

$$\text{Inhibition rate of tumor growth (\%)} = (1 - \text{average of tumor volume growth in each group/average of tumor volume growth in control group}) \times 100$$

$$\text{Rate of tumor regression (\%)} = (1 - \text{average tumor volume in each group on measuring day/average tumor volume in each group when dividing groups}) \times 100$$

The anti-tumor effect of Test Compound A1 on the next day of the final administration is shown in Table 3.

KMS-12-BM cells, OPM-2 cells, and RPMI 8226 cells which are derived from human myeloma can be purchased from, for example, ATCC, Leibniz Institute DSMZ-German Collection of Microorganisms and Cell Cultures, National Institutes of Biochemical Innovation, Health and Nutrition, JCRB cell bank, or the like.

[Table 3]

| Cell | Dosage (mg/kg) | Administration period | Inhibition or regression rate |
|---|---|---|---|
| KMS-12-BM | 8 | 21 days | 100% regression |
| OPM-2 | 8 | 7 days | 17% regression |
| RPMI 8226 | 8 | 21 days | 97% inhibition |

Test Example 4 Anti-Tumor Test on Mice Bearing Tumors of Human Myeloma Cell (2)

KMS-12-BM cells and RPMI 8226 cells which are derived from human myeloma were respectively purchased from JCRB cell bank (National Institutes of Biochemical Innovation, Health and Nutrition), and ATCC. KMS-12-BM cells were implanted to CAnN.Cg-Foxn1$^{nu}$/CrlCrlj mice (4- to 5-week-old male nude mice, Charles River Laboratories Japan, Inc.), and RPMI 8226 cells to CB17/Icr-Prkdc$^{scid}$/CrlCrlj mice (4- to 5-week-old male SCID mice, Charles River Laboratories Japan, Inc.), respectively. Each of tumor cells were prepared at 5 x $10^7$ cells/mL of a mixed solution of PBS and Matrigel (registered trademark) in 1:1 ratio, and 0.1 mL was implanted to the mice under the skin on the back (5 x $10^6$

cells/head). Dividing into groups by the tumor volume was performed when tumor cells were engrafted under the skin and the average tumor volume exceeded approximately 150 $mm^3$ (5 mice for each group). The test compound dissolved in 6% cyclodextrin aqueous solution (Sigma-Aldrich Co.) was orally administered by the dosage of 8 mg/kg or 1 mg/kg once a day. 6% Cyclodextrin aqueous solution was also orally administered for the solvent group (control group) once a day. Oral administration was performed continuously for 17 days for KMS-12-BM cells, and 18 days for RPMI 8226 cells. The inhibition rate of tumor growth and the rate of tumor regression on the next day of the final administration were calculated by using the formula below.

$$[\text{Tumor volume } (mm^3)] = [\text{major axis of tumor (mm)}] \times [\text{minor axis of tumor (mm)}]^2 \times 0.5$$

$$\text{Inhibition rate of tumor growth (\%)} = (1 - \text{average of tumor volume growth in each group/average of tumor volume growth in control group}) \times 100$$

The rate of tumor regression was calculated with respect to a group having an inhibition rate of tumor growth > 100%.

$$\text{Rate of tumor regression (\%)} = (1 - \text{average tumor volume in each group on measuring day/average tumor volume in each group when dividing groups}) \times 100$$

The anti-tumor effect of Test Compounds A1, B1, C1, and D1 on the next day of the final administration is shown in Table 4.

[Table 4]

| Test Compound | Dosage (mg/kg) | Inhibition rate or regression rate | |
|---|---|---|---|
| | | KMS-12-BM | RPMI 8226 |
| | | Administration for 17 days | Administration for 18 days |
| A1 | 8 | 99% inhibition | 99% inhibition |
| B1 | 8 | 6% regression | 4% regression |
| C1 | 1 | 68% regression | 25% regression |
| D1 | 1 | 71% inhibition | 75% inhibition |

Test Example 5 Test on Effect of Combination Use with Lenalidomide (1)

KMS-12-BM cells derived from human myeloma were prepared at 2.8 x $10^7$ cells/mL of a mixed solution of PBS and Matrigel (registered trademark) in 1:1 ratio, and 0.1 mL was implanted to CAnN.Cg-Foxn1$^{nu}$/CrlCrlj mice (4-week-old male nude mice, Charles River Laboratories Japan, Inc.) under the skin on the back (2.8 x $10^6$ cells/head). Dividing into groups by the tumor volume was performed when tumor cells were engrafted and the average tumor volume exceeded approximately 200 $mm^3$, and 5 mice were set for each group. The test compound dissolved in 6% cyclodextrin aqueous solution was orally administered by the dosage of 2 mg/kg once a day for 27 days. Lenalidomide (MedChemExpress.) was suspended in 6% cyclodextrin aqueous solution and was orally administered by the dosage of 100 mg/kg once a day for 27 days. The dosage of 2 mg/kg of the test compound and 100 mg/kg of lenalidomide were orally administered for a combination administered group once a day, respectively. 6% Cyclodextrin aqueous solution was orally administered for the solvent group (control group) once a day. The body weight and the tumor diameter were measured twice a week, and the anti-tumor effect and the effect on the body weight were examined. For calculation of the tumor volume, the formula below was used.

$$[\text{Tumor volume } (mm^3)] = [\text{major axis of tumor (mm)}] \times [\text{minor axis of tumor (mm)}]^2 \times 0.5$$

The inhibition rate of tumor growth and the rate of tumor regression were calculated from the average value of the average tumor volume according to the formula below. The rate of tumor regression was calculated with respect to a group having an inhibition rate of tumor growth > 100%.

Inhibition rate of tumor growth (%) = (1 – average of tumor volume growth in each group/average of tumor volume growth in control group) x 100

Rate of tumor regression (%) = (1 – average tumor volume in each group on measuring day/average tumor volume in each group when dividing groups) x 100

The combination effect of Test Compound A1 and lenalidomide on the next day of the final administration is shown in Table 5.

[Table 5]

| Test Compound dosage (mg/kg) | Lenalidomide dosage (mg/kg) | Inhibition or regression rate |
|---|---|---|
| No administration | 100 | 53% inhibition |
| 2 | No administration | 24% regression |
| 2 | 100 | 100% regression |

Test Example 6 Test on Effect of Combination Use with Lenalidomide (2)

KMS-12-BM cells derived from human myeloma were prepared at 5 x $10^7$ cells/mL of a mixed solution of PBS and Matrigel (registered trademark) in 1:1 ratio, and 0.1 mL was implanted to CAnN.Cg-Foxn1$^{nu}$/CrlCrlj mice (4- to 5-week-old male nude mice, Charles River Laboratories Japan, Inc.) under the skin on the back (5 x $10^6$ cells/head). Dividing into groups by the tumor volume was performed when tumor cells were engrafted under the skin and the average tumor volume exceeded approximately 200 $mm^3$. A total 10 groups of a solvent + solvent (control group), a Compound A1 + solvent, a Compound B1 + solvent, a Compound C1 + solvent, a Compound D1 + solvent, a solvent + lenalidomide, a Compound A1 + lenalidomide, a Compound B1 + lenalidomide, a Compound C1 + lenalidomide, and a Compound D1 + lenalidomide administered group were set as a test group, and 5 mice were set for each group. The test compound dissolved in 6% cyclodextrin aqueous solution was orally administered by the dosage of 8 mg/kg or 1 mg/kg once a day for 14 days continuously. Lenalidomide (MedChemExpress.) was suspended in 6% cyclodextrin aqueous solution and was orally administered by the dosage of 100 mg/kg once a day for 14 days continuously. The inhibition rate of tumor growth and the rate of tumor regression in each group on the next day of the final administration were calculated by using the formula below.

$$[\text{Tumor volume } (mm^3)] = [\text{major axis of tumor } (mm)] \times [\text{minor axis of tumor } (mm)]^2 \times 0.5$$

Inhibition rate of tumor growth (%) = (1 – average of tumor volume growth in each group/average of tumor volume growth in control group) x 100

The rate of tumor regression was calculated with respect to a group having an inhibition rate of tumor growth > 100%.

Rate of tumor regression (%) = (1 – average tumor volume in each group on measuring day/average tumor volume in each group when starting administration) x 100

The inhibition rate of tumor growth and the rate of tumor regression in each group on the next day of the final administration is shown in Table 6.

[Table 6]

| Test Compound | Test Compound dosage (mg/kg) | Lenalidomide dosage (mg/kg) | Inhibition rate or regression rate |
|---|---|---|---|
| A1 | 8 | 0 | 100% inhibition |
| B1 | 8 | 0 | 20% regression |
| C1 | 1 | 0 | 40% regression |
| D1 | 1 | 0 | 61% inhibition |
| Solvent | 0 | 100 | 21% inhibition |
| A1 | 8 | 100 | 39% regression |
| B1 | 8 | 100 | 42% regression |
| C1 | 1 | 100 | 64% regression |
| D1 | 1 | 100 | 74% inhibition |

From the above results, it was confirmed that Compound A, Compound B, Compound C, and Compound D which are active ingredients of a pharmaceutical composition of the present invention inhibit mitochondrial Complex I and have the effect of activating AMPK. In addition, it was confirmed that the compounds have an anti-tumor effect with respect to mice bearing tumors of human myeloma. Furthermore, it was confirmed that the combination use of lenalidomide and Compound A, Compound B, Compound C, and Compound D exhibited a stronger anti-tumor effect than each single administration thereof.

Accordingly, a compound selected from Compound A, Compound B, Compound C, and Compound D or a pharmaceutically acceptable salt thereof can be used for treating multiple myeloma.

A pharmaceutical composition comprising a compound selected from Compound A, Compound B, Compound C, and Compound D or a pharmaceutically acceptable salt thereof as an active ingredient may include excipients as an arbitrary additive, or can be prepared by methods which are commonly used, using excipients commonly used in this field, that is, pharmaceutical excipients, pharmaceutical carrier, or the like.

Administration may be any form of oral administration by a tablet, a pill, a capsule, a granule, powder, a liquid, and the like, or parenteral administration by intra-articular, intravenous, intramuscular, and the like injections, a suppository, a transdermal solution, an ointment, a transdermal patch, a transmucosal solution, a transmucosal patch, and the like.

As a solid composition for the oral administration, a tablet, powder, a granule, and the like is used. In such solid composition, one or two or more kinds of active ingredients are mixed with at least one inert excipient. The composition may contain an inert additive, for example, a lubricant, a disintegrant, a stabilizer, a solubilizer, and the like by commonly used methods. The tablet or the pill may be coated with a film of sugar or a stomach-soluble, or enteric-soluble substance, if necessary.

A liquid composition for the oral administration includes an emulsion, a solution preparation, a suspension, a syrup or an elixir, and the like which is pharmaceutically acceptable, and includes a generally used inert diluent, for example, purified water or ethanol. The liquid composition may contain adjuvants such as a solubilizing agent, a wetting agent, and a suspension, a sweetener, a flavor, an aromatic, or a preservative in addition to the inert diluent.

The injection for the parenteral administration includes a sterile aqueous or non-aqueous solution preparation, a suspension or an emulsion. As the aqueous solvent, for example, distilled water for injection or physiological saline is included. As the non-aqueous solvent, for example, alcohols such as ethanol are included. Such a composition may further include a tonicity agent, a preservative, a wetting agent, an emulsifier, a dispersant, a stabilizer, or a solubilizer. These are sterilized by, for example, filtration through a bacteria-retaining filter, mixing of a germicide, or irradiation. In addition, these can also be used in a manner in which a sterile solid composition is prepared, and is dissolved or suspended in sterile water or a sterile solvent for injection before being used.

As an external application, an ointment, a plaster, a cream, a jelly, a poultice, a spray, a lotion, and the like is included. A generally used ointment base, lotion base, aqueous or non-aqueous solution, suspension, emulsion, and the like is contained.

The transmucosal agent such as a transnasal agent and the like is used in a solid, liquid, or semi-solid form, and can be prepared according to methods known in the related art. For example, a known excipient, a pH adjuster, a preservative, a surfactant, a lubricant, a stabilizer, a thickener, and the like may be suitably added. In administration, it is possible to use an appropriate device for inhalation or insufflation. For example, by using a known device such as a

metered dose inhaler device and the like, or a nebulizer, the administration can be performed as a powder of a compound alone or of a prescribed mixture, or as a solution or a suspension in combination with a carrier which is pharmaceutically acceptable. A dry powder inhaler and the like may be an inhaler for single or multiple administration, and it is possible to use a dry powder or a powder-containing capsule. Alternatively, this may be in a form of a pressurized aerosol spray and the like that uses an appropriate propellant, for example, a suitable gas such as chlorofluoroalkane or carbon dioxide, and the like.

In a case of normal oral administration, a daily dose is approximately 0.001 to 100 mg/kg of body weight, preferably 0.1 to 30 mg/kg, and more preferably 0.1 to 10 mg/kg, and this dose is administered at once or in 2 to 4 divided doses. In a case of an intravenous administration, approximately 0.0001 to 10 mg/kg of body weight is suitable for a daily dose, and this dose is administered at once or in multiple divided doses per day. In addition, as the transmucosal agent, approximately 0.001 to 100 mg/kg of body weight is administered at once or in multiple divided doses per day. The dose is appropriately determined according to individual cases in consideration of symptoms, age, gender, or the like.

The amount differs depending on the type of administration route, dosage form, administration site, excipients, and additives, but the pharmaceutical composition of the present invention contains 0.01 to 100% by weight, and in a certain embodiment, 0.01 to 50% by weight of a compound selected from Compound A, Compound B, Compound C, and Compound D or a pharmaceutically acceptable salt thereof which are active ingredients.

There is a possibility that the pharmaceutical composition of the present invention can be used together with various agents as well as lenalidomide, for treating diseases which are believed to exhibit effectiveness with respect to multiple myeloma. For use in combination, co-administration or separate administration in succession may be performed, or administration may be performed at a desired time interval. When performing the co-administration, these may be a combination agent or may be formulated separately.

Examples

Hereinafter, preparation methods for Compound A, Compound B, Compound C, and Compound D will be described in detail based on examples. In addition, preparation methods for starting compounds thereof will be described in Preparation Examples. In addition, the preparation methods for Compound A, Compound B, Compound C, and Compound D are not limited to the preparation methods in the specific examples shown below, and the compounds can also be prepared by using another combination of the preparation methods, or a method obvious to those skilled in the art.

In the present specification, naming a software such as ACD/Name (registered trademark, manufactured by Advanced Chemistry Development, Inc.) or the like is used in naming of compounds in some cases.

In addition, for the sake of convenience, a concentration mol/l is expressed by M. For example, a 1 M aqueous sodium hydroxide solution means a 1 mol/l aqueous sodium hydroxide solution.

The powder X-ray diffraction was measured using RINT-TTRII (manufactured by RIGAKU Corporation) under the conditions of tube: Cu, tube current: 300 mA, tube voltage: 50 kV, sampling width: 0.020°, scanning speed: 4°/min, wavelength: 1.54056 A, and measurement diffraction angle range (2θ): 2.5° to 40°. Handling of a device including a data process was in accordance with the methods and procedures instructed on each device.

Each crystal was characterized by a powder X-ray diffraction pattern, respectively, but judging from the nature of data of the powder X-ray diffraction, the crystal lattice distance and the overall pattern are important in determining the identity of the crystal, and the diffraction angle and diffraction intensity are not to be strictly interpreted since these may vary slightly in accordance with the direction of crystal growth, the particle size, and measurement conditions. The diffraction angle (2θ(°)) of powder X-ray diffraction patterns is interpreted in consideration of an error range that is generally acceptable in the measuring method, and the error range is ±0.2° in a certain embodiment.

Preparation Example 1

N-[3-(dimethylamino)propyl]-N'-ethylcarbodiimide hydrochloride (1.2 g) was added to a mixture of 5-bromo-1H-benzimidazol-2-carboxylic acid (1.0 g), 1-[4-(trifluoromethyl)benzyl]piperazine (1.0 g), 1H-benzotriazol-1-ol (840 mg), and N,N-dimethylformamide (10 ml: hereinafter, abbreviated as DMF), followed by stirring at room temperature overnight. A saturated aqueous sodium hydrogen carbonate solution was added to the reaction mixture, followed by stirring at room temperature for 1 hour, and the resulting solid was collected by filtration, followed by drying under reduced pressure. The obtained solid was dissolved in a mixture of chloroform (100 ml) and ethanol (1 ml) while heating to reflux. The mixture was cooled to room temperature and then hexane (100 ml) was added thereto. The resulting solid was collected by filtration, followed by drying under reduced pressure, thereby obtaining (5-bromo-1H-benzimidazol-2-yl){4-[4-(trifluoromethyl)benzyl]piperazin-1-yl}methanone (1.4 g) as a solid.

Preparation Example 2

A mixture of (5-bromo-1H-benzimidazol-2-yl){4-[4-(trifluoromethyl)benzyl)piperazin-1-yl}methanone (1.2 g), tert-butyl 4-(4,4,5,5-tetramethyl-1,3,2-dioxaborolan-2-yl)-3,6-dihydropyridine-1(2H)-carboxylate (1.6 g), tetrakis(triphenylphosphine)palladium (590 mg), sodium carbonate (2.2 g), dioxane (40 ml), and water (10 ml) was stirred at 95°C for 24 hours in an argon atmosphere, and then cooled to room temperature. Water was added to the reaction mixture, and extraction was carried out using ethyl acetate. After the organic layer was dried over anhydrous sodium sulfate, the desiccant was removed, and then the solvent was evaporated under reduced pressure. The obtained residue was purified by silica gel column chromatography (chloroform-methanol), thereby obtaining tert-butyl 4-[2-({4-[4-(trifluoromethyl)benzyl]piperazin-1-yl}carbonyl)-1H-benzimidazol-5-yl]-3,6-dihydropyridine-1(2H)-carboxylate (1.2 g) as an oily material.

Preparation Example 3

To an ethanol (40 ml) solution of tert-butyl 4-[2-({4-[4-(trifluoromethyl)benzyl]piperazin-1-yl}carboxyl)-1H-benzimidazol-5-yl]-3,6-dihydropyridine-1(2H)-carboxylate (1.4 g) was added 10% palladium-activated charcoal (approximately 50% water-containing product, 500 mg), followed by stirring at room temperature for 6 hours in a hydrogen atmosphere. The insoluble material was removed, and then the solvent was evaporated under reduced pressure. To an ethanol (40 ml) solution of the obtained residue was added 10% palladium-activated charcoal (approximately 50% water-containing product, 500 mg), followed by stirring at room temperature for 4 hours in a hydrogen atmosphere of 3.0 kgf/$cm^2$. The insoluble material was removed, and then the solvent was evaporated under reduced pressure. To a methanol (41 ml) solution of the obtained residue was added 20% palladium hydroxide-activated charcoal (approximately 50% water-containing product, 800 mg), followed by stirring at room temperature for 24 hours in a hydrogen atmosphere of 3.0 kgf/$cm^2$. The insoluble material was removed, and then the solvent was evaporated under reduced pressure. The obtained residue was purified by silica gel column chromatography (chloroform-methanol), thereby obtaining tert-butyl 4-[2-({4-[4-(trifluoromethyl)benzyl]piperazin-1-yl}carboxyl)-1H-benzimidazol-5-yl]piperidine-1-carboxylate (1.1 g) as an oily material.

Preparation Example 4

A 4 M hydrogen chloride/ethyl acetate solution (5 ml) was added to an ethyl acetate (30 ml) solution of tert-butyl 4-[2-({4-[4-(trifluoromethyl)benzyl]piperazin-1-yl}carbonyl)-1H-benzimidazol-5-yl]piperidine-1-carboxylate (1.1 g), the reaction mixture was stirred at room temperature for 6 hours, and then left to stand overnight. The solvent was evaporated under reduced pressure, and then ethyl acetate and hexane were added to the obtained residue. The resulting solid was collected by filtration, followed by drying under reduced pressure, thereby obtaining hydrochloride of [5-(piperidin-4-yl)-1H-benzimidazol-2-yl]{4-[4-(trifluoromethyl)benzyl]piperazin-1-yl}methanone (740 mg: a molar ratio to hydrogen chloride was undetermined) as a solid.

Preparation Example 5

Trifluoroacetic acid (1 ml) was added to a dichloromethane (2 ml) solution of tert-butyl 4-[2-({4-[4-(trifluoromethyl)benzyl]piperazin-1-yl}carbonyl)-1H-benzimidazol-5-yl]piperidine-1-carboxylate (270 mg), followed by stirring at room temperature for 30 minutes. A saturated aqueous sodium hydrogen carbonate solution was added to the reaction mixture, and extraction was carried out using chloroform. The organic layer was washed with a saturated aqueous sodium chloride solution and then dried over anhydrous sodium sulfate. The desiccant was removed, and then the solvent was evaporated under reduced pressure. The obtained residue was purified by amino silica gel column chromatography (chloroform-methanol), thereby obtaining [5-(piperidin-4-yl)-1H-benzimidazol-2-yl]{4-[4-(trifluoromethyl)benzyl]piperazin-1-yl}methanone (150 mg) as an amorphous material.

Preparation Example 6

A mixture of 6-bromo-1H-indole-2-carboxylate (1.0 g), N-[3-(dimethylamino)propyl]-N'-ethylcarbodiimide hydrochloride (1.1 g), 1H-benzotriazol-1-ol (770 mg), and dichloromethane (15 ml) was stirred at room temperature for 10 minutes. To the reaction mixture were added 4-(piperazin-1-ylmethyl)benzonitrile dihydrochloride (1.2 g) and N,N-diisopropylethylamine (1.6 ml), followed by stirring at room temperature overnight. A saturated aqueous sodium hydrogen carbonate solution was added to the reaction mixture, extraction was carried out using chloroform, and then extraction was carried out using chloroform-methanol. After the organic layer was dried over anhydrous magnesium sulfate, the desiccant was removed, and then the solvent was evaporated under reduced pressure. The obtained residue was purified by silica gel column chromatography (chloroform-methanol), thereby obtaining 4-({4-[(6-bromo-1H-indol-2-yl)carbonyl]piperazin-1-yl}methyl)benzonitrile (1.1 g) as a solid.

Preparation Example 7

A mixture of 4-({4-[(6-bromo-1H-indol-2-yl)carbonyl]piperazin-1-yl}methyl)benzonitrile (1.1 g), tert-butyl 4-(4,4,5,5-tetramethyl-1,3,2-dioxaborolan-2-yl)-3,6-dihydropyridine-1(2H)-carboxylate (1.6 g), tetrakis(triphenylphosphine)palladium (480 mg), sodium carbonate (790 mg), dioxane (18 ml), and water (1.8 ml) was stirred at 100°C overnight in an argon atmosphere, and then cooled to room temperature. Water was added to the reaction mixture, and extraction was carried out using chloroform. The organic layer was dried over anhydrous sodium sulfate, the desiccant was removed, and then the solvent was evaporated under reduced pressure. The obtained residue was purified by silica gel column chromatography (chloroform-methanol, and then hexane-ethyl acetate), and the obtained solid was washed with diisopropyl ether, thereby obtaining tert-butyl 4-(2-{[4-(4-cyanobenzyl)piperazin-1-yl]carbonyl}-1H-indol-6-yl)-3,6-dihydropyridine-1(2H)-carboxylate (470 mg) as a solid.

Preparation Example 8

To a mixture of tert-butyl 4-(2-{[4-(4-cyanobenzyl)piperazin-1-yl]carbonyl}-1H-indol-6-yl)-3,6-dihydropyridine-1(2H)-carboxylate (470 mg), tetrahydrofuran (hereinafter, abbreviated as THF) (14 ml), and ethanol (3 ml) was added 10% palladium-activated charcoal (approximately 50% water-containing product, 230 mg), followed by stirring at room temperature for 2 hours in a hydrogen atmosphere. After the insoluble material was removed, the solvent was evaporated under reduced pressure, and 10% palladium-activated charcoal (approximately 50% water-containing product, 230 mg) was added to a mixture of the obtained residue, THF (14 ml), and ethanol (3 ml), followed by stirring at room temperature overnight in a hydrogen atmosphere. After the insoluble material was removed, the solvent was evaporated under reduced pressure. After 20% palladium hydroxide-activated charcoal (approximately 50% water-containing product, 230 mg) was added to a mixture of the obtained residue, THF (14 ml), and ethanol (3 ml), followed by stirring at room temperature for 4 hours in a hydrogen atmosphere of 3.0 $kgf/cm^2$, and being left to stand for 3 days. The insoluble material was removed, and then the solvent was evaporated under reduced pressure. The obtained residue was purified by amino silica gel column chromatography (chloroform-methanol), thereby obtaining tert-butyl 4-[2-(piperazin-1-ylcarbonyl)-1H-indol-6-yl]piperidine-1-carboxylate (360 mg) as an oily material.

Preparation Example 9

Sodium triacetoxyborohydride (360 mg) was added to a mixture of tert-butyl 4-[2-(piperazin-1-ylcarbonyl)-1H-indol-6-yl]piperidine-1-carboxylate (350 mg), 4-formylbenzonitrile (140 mg), and dichloromethane (3 ml), followed by stirring at room temperature for 1.5 hours. A saturated aqueous sodium hydrogen carbonate solution and methanol were added to the reaction mixture, and extraction was carried out using chloroform. The organic layer was dried over anhydrous sodium sulfate, the desiccant was removed, and then the solvent was evaporated under reduced pressure. The obtained residue was purified by silica gel column chromatography (chloroform-methanol), thereby obtaining tert-butyl 4-(2-{[4-(4-cyanobenzyl)piperazin-1-yl]-carbonyl}-1H-indol-6-yl)piperidine-1-carboxylate (450 mg) as an oily material.

Preparation Example 10

Sodium hydride (containing approximately 45% of a liquid paraffin, 40 mg) was added to a DMF (4 ml) solution of tert-butyl 4-(2-{[4-(4-cyanobenzyl)piperazin-1-yl]-carbonyl}-1H-indol-6-yl)piperidine-1-carboxylate (450 mg) under ice-cooling, followed by stirring at room temperature for 1 hour. Methyl iodide (58 μl) was added to the reaction mixture at room temperature, followed by stirring at room temperature for 1 hour. A saturated aqueous ammonium chloride solution and water were added to the reaction mixture, and extraction was carried out using ethyl acetate. The organic layer was washed with water and a saturated aqueous sodium chloride solution in this order, and then dried over anhydrous sodium sulfate. The desiccant was removed, and then the solvent was evaporated under reduced pressure. The obtained residue was purified by silica gel column chromatography (chloroform-methanol), thereby obtaining tert-butyl 4-(2-{[4-(4-cyanobenzyl)piperazin-1-yl]carbonyl}-1-methyl-1H-indol-6-yl)piperidine-1-carboxylate (200 mg) as an oily material.

Preparation Example 11

Trifluoroacetic acid (500 μl) was added to a dichloromethane (1 ml) solution of tert-butyl 4-(2-{[4-(4-cyanobenzyl)piperazin-1-yl]carbonyl}-1-methyl-1H-indol-6-yl)piperidine-1-carboxylate (200 mg) at room temperature, followed by stirring at room temperature for 2 hours. The solvent was evaporated under reduced pressure, a saturated aqueous sodium hydrogen carbonate solution and water were added to the obtained residue, and then extraction was carried out

using chloroform. The organic layer was dried over anhydrous sodium sulfate, the desiccant was removed, and then the solvent was evaporated under reduced pressure. The obtained residue was purified by amino silica gel column chromatography (chloroform-methanol), thereby obtaining 4-[(4-{[1-methyl-6-(piperidin-4-yl)-1H-indol-2-yl]carbonyl)piperazin-1-yl)methyl]benzonitrile (120 mg) as a solid.

Preparation Example 12

Sodium borohydride (2.6 g) was added in portions plural times to a methanol (100 ml) solution of ethyl 5-ethoxypyrazine-2-carboxylate (4.5 g) under ice-cooling, followed by stirring at room temperature for 6 hours. 1 M hydrochloric acid was added to the reaction mixture so that pH became 4, followed by stirring at room temperature for 15 minutes. AIM aqueous sodium hydroxide solution was added to the mixture so that pH became 9, and then extraction was carried out using chloroform. The organic layer was dried over anhydrous sodium sulfate, the desiccant was removed, and the solvent was evaporated under reduced pressure. The obtained residue was purified by silica gel column chromatography (hexane-ethyl acetate), thereby obtaining (5-ethoxypyrazin-2-yl)methanol (2.6 g) as an oily material.

Preparation Example 13

Thionyl chloride (200 μl) was added to a dichloromethane (3 ml) solution of (5-ethoxypyrazin-2-yl)methanol (150 mg) under ice-cooling, followed by stirring at room temperature for 30 minutes and concentration under reduced pressure, thereby obtaining 2-(chloromethyl)-5-ethoxypyrazine (160 mg) as an oily material.

Preparation Example 14

A mixture of ethyl 5-bromo-1H-indole-2-carboxylate (5.2 g), tert-butyl 4-(4,4,5,5-tetramethyl-1,3,2-dioxaborolan-2-yl)-3,6-dihydropyridine-1(2H)-carboxylate (13 g), tetrakis(triphenylphosphine)palladium (5.3 g), 2 M aqueous sodium carbonate solution (28 ml), and dioxane (110 ml) was stirred at 95°C for 17 hours in an argon atmosphere, and then cooled to room temperature. Water was added to the reaction mixture, and extraction was carried out using chloroform. The organic layer was dried over anhydrous sodium sulfate, the desiccant was removed, and then the solvent was evaporated under reduced pressure. The obtained residue was purified by silica gel column chromatography (hexane-ethyl acetate, and then chloroform-methanol) and amino silica gel column chromatography (hexane-ethyl acetate). Hexane-diisopropyl ether was added to the obtained solid (6.5 g), and powderization was carried out. The solid was collected by filtration, followed by drying under reduced pressure, thereby obtaining ethyl 5-[1-(tert-butoxycarbonyl)-1,2,3,6-tetrahydropyridin-4-yl]-1H-indole-2-carboxylate (5.0 g) as a solid.

Preparation Example 15

To a mixture of ethyl 5-[1-(tert-butoxycarbonyl)-1,2,3,6-tetrahydropyridin-4-yl]-1H-indole-2-carboxylate (5.0 g), ethanol (55 ml), and THF (55 ml) was added 20% palladium hydroxide-activated charcoal (approximately 50% water-containing product, 1.0 g), followed by stirring at room temperature for 3 hours in a hydrogen atmosphere. The insoluble material was removed, and then the solvent was evaporated under reduced pressure. The obtained residue was purified by amino silica gel column chromatography (hexane-ethyl acetate), thereby obtaining ethyl 5-[1-(tert-butoxycarbonyl)piperidin-4-yl]-1H-indole-2-carboxylate (4.8 g) as a solid.

Preparation Example 16

Dimethyl sulfate (1.8 ml) was added to a mixture of ethyl 5-[1-(tert-butoxycarbonyl)piperidin-4-yl]-1H-indole-2-carboxylate (4.8 g), cesium carbonate (7.0 g), and acetonitrile (70 ml), followed by stirring at 75°C for 2 hours, and then cooled to room temperature. After ethyl acetate was added to the reaction mixture, washing was performed with water and a saturated aqueous sodium chloride solution in this order. The organic layer was dried over anhydrous sodium sulfate, the desiccant was removed, and then the solvent was evaporated under reduced pressure, thereby obtaining ethyl 5-[1-(tert-butoxycarbonyl)piperidin-4-yl]-1-methyl-1H-indole-2-carboxylate (5.7 g) as an oily material.

Preparation Example 17

AIM aqueous sodium hydroxide solution (23 ml) was added to a mixture of ethyl 5-[-(tert-butoxycarbonyl)piperidin-4-yl]-1-methyl-1H-indole-2-carboxylate (5.7 g), dioxane (23 ml), and ethanol (23 ml), followed by stirring at 60°C overnight, and then cooled to room temperature. 1 M hydrochloric acid (23 ml) was added to the reaction mixture under ice-cooling, and extraction was carried out using chloroform. After the organic layer was dried over anhydrous sodium

sulfate, the desiccant was removed, and the solvent was evaporated under reduced pressure, thereby obtaining 5-[1-(tert-butoxycarbonyl)piperidin-4-yl]-1-methyl-1H-indole-2-carboxylate (4.8 g) as an amorphous material.

Preparation Example 18

To a mixture of 5-[1-(tert-butoxycarbonyl)piperidin-4-yl]-1-methyl-1H-indole-2-carboxylate (4.8 g), 1H-benzotriazol-1-ol (1.9 g), N,N-diisopropylethylamine (6.8 ml), and dichloromethane (55 ml) were added 4-(piperazin-1-ylmethyl)benzonitrile dihydrochloride (4.0 g) and N-[3-(dimethylamino)propyl]-N'-ethylcarbodiimide hydrochloride (3.1 g), followed by stirring at room temperature for 2 hours. A saturated aqueous sodium hydrogen carbonate solution and water were added to the reaction mixture, and extraction was carried out using chloroform. The organic layer was dried over anhydrous sodium sulfate, the desiccant was removed, and then the solvent was evaporated under reduced pressure. The obtained residue was purified by silica gel column chromatography (hexane-ethyl acetate), thereby obtaining tert-butyl 4-(2-{[4-(4-cyanobenzyl)piperazin-1-yl]carbonyl}-1-methyl-1H-indol-5-yl)piperidine-1-carboxylate (6.8 g) as an amorphous material.

Preparation Example 19

Trifluoroacetic acid (5 ml) was added to a dichloromethane (10 ml) solution of tert-butyl 4-(2-{[4-(4-cyanobenzyl)piperazin-1-yl]carbonyl}-1-methyl-1H-indol-5-yl)piperidine-1-carboxylate (6.8 g) at room temperature, followed by stirring at room temperature for 2 hours. The solvent was evaporated under reduced pressure, a saturated aqueous sodium hydrogen carbonate solution was added to the obtained residue, and then extraction was carried out using chloroform. The organic layer was dried over anhydrous sodium sulfate, the desiccant was removed, and then the solvent was evaporated under reduced pressure, thereby obtaining 4-[(4-{[1-methyl-5-(piperidin-4-yl)-1H-indol-2-yl]carbonyl}piperazin-1-yl)methyl]benzonitrile (7.1 g) as an amorphous material.

Example 1

A mixture of hydrochloride (200 mg) of [5-(piperidin-4-yl)-1H-benzimidazol-2-yl]{4-[4-(trifluoromethyl)benzyl]piperazin-1-yl}methanone, 6-methoxynicotinaldehyde (100 mg), triethylamine (140 $\mu$l), acetic acid (100 $\mu$l), and dichloromethane (4 ml) was stirred at room temperature for 10 minutes. Sodium triacetoxyborohydride (580 mg) was added to the reaction mixture at room temperature, followed by stirring at room temperature for 2 hours, and being left to stand at room temperature overnight. A saturated aqueous sodium hydrogen carbonate solution was added to the reaction mixture, and extraction was carried out using chloroform. After the organic layer was dried over anhydrous sodium sulfate, the desiccant was removed, and then the solvent was evaporated under reduced pressure. The obtained crude product was purified by amino silica gel column chromatography (chloroform-methanol), tosic acid monohydrate (69 mg) was added to an acetone solution of the obtained oily material (Compound A, 110 mg), and then the solvent was evaporated under reduced pressure. Ethanol (3 ml) and diisopropyl ether (20 ml) were added to the obtained residue, followed by stirring at room temperature. The resulting solid was collected by filtration, and then dried under reduced pressure, thereby obtaining ditosylate salts of (5-{1-[(6-methoxypyridin-3-yl)methyl]piperidin-4-yl}-1H-benzimidazol-2-yl){4-[4-(trifluoromethyl)benzyl]piperazin-1-yl}methanone (Compound A) (180 mg) as an amorphous material. In addition, after stirring a mixture of Compound A (200 mg) prepared in the same manner as above and acetonitrile (10 ml) at 95°C for 30 minutes, tosic acid monohydrate (130 mg) was added thereto. The mixture was cooled to room temperature while stirring, and then stirred at room temperature for 7 days. The resulting solid was collected by filtration, and then dried under reduced pressure, thereby obtaining ditosylate salts of (5-{1-[(6-methoxypyridin-3-yl)methyl]piperidin-4-yl} -1 H-benzimidazol-2-yl) {4-[4-(trifluoromethyl)benzyl]piperazin-1-yl}methanone (Compound A) (300 mg) as a crystal. The powder X-ray diffraction data of this crystal is shown in Table 12 described below.

Example 2

To a mixture of [5-(piperidin-4-yl)-1H-benzimidazol-2-yl]{4-[4-(trifluoromethyl)benzyl)piperazin-1-yl}methanone (47 mg), N,N-diisopropylethylamine (68 $\mu$l), acetonitrile (1ml), and DMF (1 ml) was added 2-(chloromethyl)-5-methoxypyrazine (16 mg), followed by stirring at room temperature for 5 days. Water was added to the reaction mixture, and extraction was carried out using ethyl acetate. The organic layer was dried over anhydrous sodium sulfate, the desiccant was removed, and then the solvent was evaporated under reduced pressure. The obtained crude product was purified by silica gel column chromatography (chloroform-methanol). Tosic acid monohydrate (24 mg) and ethyl acetate (3 ml) were added to an acetone (2 ml) solution of the obtained oily material (Compound B, 38 mg), followed by stirring at room temperature overnight. The resulting solid was collected by filtration, and then dried under reduced pressure, thereby obtaining ditosylate salts of (5-{1-[(5-methoxypyrazin-2-yl)methyl]piperidin-4-yl}-1H-benzimidazol-2-yl){4-[4-(trifluoromethyl)benzyl)piperazin-1-yl}methanone (Compound B) (53 mg) as a solid. In addition, after stirring a mixture of Compound B (200 mg), prepared in the same manner as above, acetone (18 ml), and acetonitrile (3 ml) at 80°C, tosic acid monohydrate (130 mg) was added thereto. The mixture was cooled to room temperature while stirring, and then stirred at room temperature for 72 hours. The resulting solid was collected by filtration, and then dried under reduced pressure, thereby obtaining ditosylate salts of (5-{1-[(5-methoxypyrazin-2-yl)methyl]piperidin-4-yl}-1H-benzimidazol-2-yl){4-[4-(trifluoromethyl)benzyl]piperazin-1-yl}methanone (Compound B) (300 mg) as a crystal. The powder X-ray diffraction data of this crystal is shown in Table 12 described below.

Example 3

An acetonitrile (500 μl) solution of 2-(chloromethyl)-5-ethoxypyrazine (53 mg) was added to a mixture of 4-[(4-{1-methyl-6-(piperidin-4-yl)-1H-indol-2-yl]carbonyl}piperazin-1-yl)methyl]benzonitrile (120 mg), N,N-diisopropylethylamine (160 μl), and acetonitrile (1 ml), followed by stirring at room temperature overnight. After the solvent of the reaction mixture was evaporated under reduced pressure, the obtained residue was purified by silica gel column chromatography (chloroform-methanol) and DIOL silica gel column chromatography (hexane-ethyl acetate). After stirring a mixture of the obtained solid (110 mg) and acetone (3 ml) at 80°C, tosic acid monohydrate (68 mg) was added thereto. The mixture was cooled to room temperature while stirring, and then stirred at room temperature overnight. The resulting solid was collected by filtration, and then dried under reduced pressure, thereby obtaining ditosylate salts of 4-({4-[(6-{1-[(5-ethoxypyrazin-2-yl)methyl]piperidin-4-yl}-1-methyl-1H-indol-2-yl)carbonyl]piperazin-1-yl}methyl)benzonitrile (Compound C) (130 mg) as a crystal. The powder X-ray diffraction data of this crystal is shown in Table 12 described below.

Example 4

A dichloromethane (5 ml) solution of 2-(chloromethyl)-5-methoxypyrazine (760 mg) was added to a mixture of 4-[(4-{[1-methyl-5-(piperidin-4-yl)-1H-indol-2-yl]carbonyl}piperazin-1-yl)methyl]benzonitrile (2.0 g), N,N-diisopropylethylamine (2.7 ml), and acetonitrile (10 ml), followed by stirring at room temperature for 5 days. After the solvent of the reaction mixture was evaporated under reduced pressure, the obtained residue was purified by silica gel column chromatography (chloroform-methanol) and amino silica gel column chromatography (hexane-ethyl acetate, and then chloroform-methanol), thereby obtaining amorphous material (1.4 g). After the obtained amorphous material (1.2 g) was purified by silica gel column chromatography (chloroform-methanol), a mixture of the obtained solid (760 mg) and acetone (100 ml) was heated and stirred at 80°C for 30 minutes, and then tosic acid monohydrate (510 mg) was added thereto. The mixture was cooled to room temperature while stirring, and then stirred at room temperature for 4 hours. The resulting solid was collected by filtration, and then dried under reduced pressure, thereby obtaining ditosylate salts of 4-({4-[(5-{1-[(5-methoxypyrazin-2-yl)methyl]piperidin-4-yl}-1-methyl-1H-indol-2-yl)carbonyl]piperazin-1-yl}methyl)benzonitrile (Compound D) (1.1 g) as a crystal. The powder X-ray diffraction data of this crystal is shown in Table 12 described below.

The structure and physicochemical data of the compounds of the preparation examples and the compounds of examples are shown in Tables 7 to 12 described below.

The following abbreviations may be used in Tables 7 to 12 described below.

Pre: preparation example No, Ex: example No, Str: chemical structure formula, Dat: physicochemical data, ESI+: m/z value in mass spectrometry (ionization method ESI, $[M+H]^+$ unless otherwise specified), ESI-: m/z value in mass spectrometry (ionization method ESI, $[M-H]^-$ unless otherwise specified), APCI/ESI: APCI/ESI-MS (atmospheric pressure chemical ionization method APCI, APCI/ESI refers to the simultaneous measurement of APCI and ESI, and APCI/ESI+ is $[M+H]^+$), NMR1: δ (ppm) of peak in $^1$H-NMR in $CD_3OD$, NMR2: δ (ppm) of peak in $^1$H-NMR in DMSO-$d_6$, Me: methyl, Et: ethyl, Boc: tert-butoxycarbonyl, and 2θ(°): diffraction angle of powder X-ray diffraction.

Furthermore, in the chemical structure formulas, xHCl indicates that the compound is a hydrochloride, but the molar ratio to hydrogen chloride is undetermined, and 2TsOH indicates that the compound is a ditosylate salt, respectively.

[Table 7]

| Pre | Str | Dat |
|---|---|---|
| 1 | 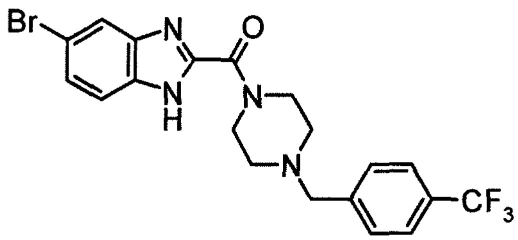 | ESI+: 467, 469 |
| 2 | 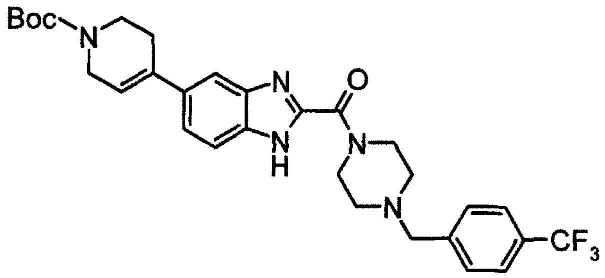 | ESI+: 570 |
| 3 | 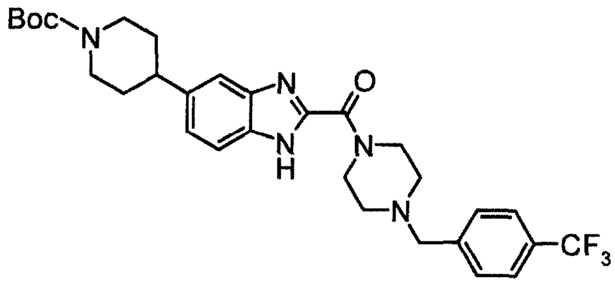 | ESI+: 572 |
| 4 | 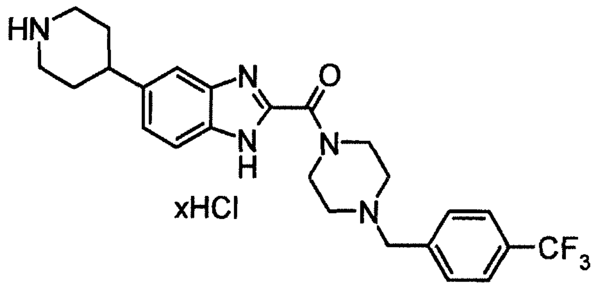 | ESI+: 472 |
| 5 | 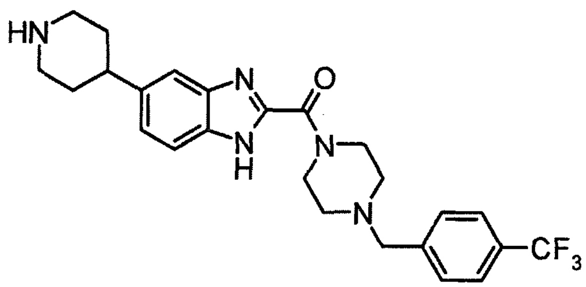 | APCI/ESI+: 472 |

[Table 8]

| Pre | Str | Dat |
|---|---|---|
| 6 | 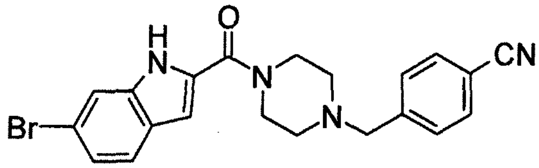 | ESI+: 423, 425 |
| 7 | 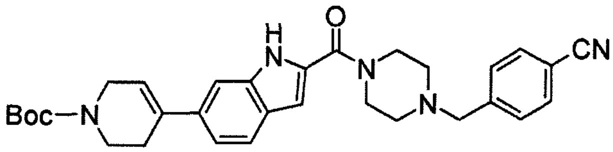 | ESI+: 526 |
| 8 | 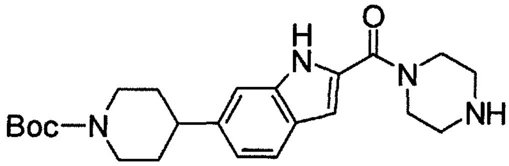 | ESI-: 411 |
| 9 | 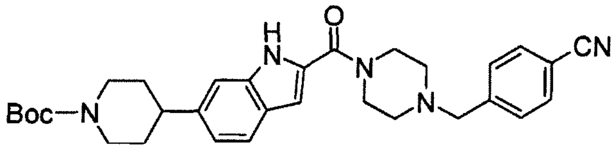 | ESI-: 526 |
| 10 | 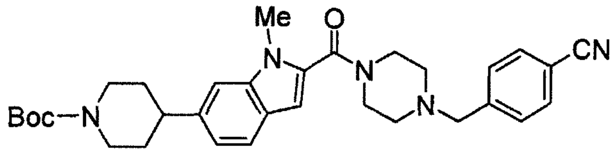 | ESI+: 542 |
| 11 | 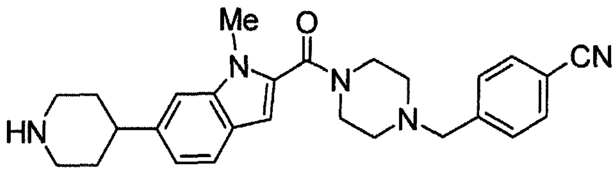 | ESI+: 442 |
| 12 | 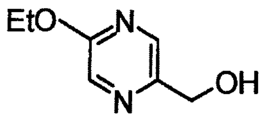 | ESI+: 155 |

[Table 9]

| Pre | Str | Dat |
|---|---|---|
| 13 | 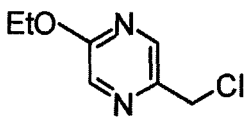 | ESI+: 173, 175 |

(continued)

| Pre | Str | Dat |
|---|---|---|
| 14 | 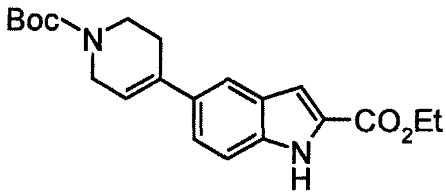 | ESI-: 369 |
| 15 | 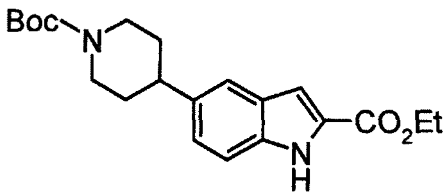 | ESI+: 373 |
| 16 | 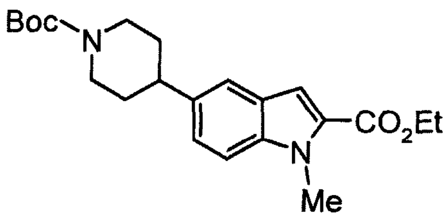 | ESI+: 409 [M+Na]+ |
| 17 | 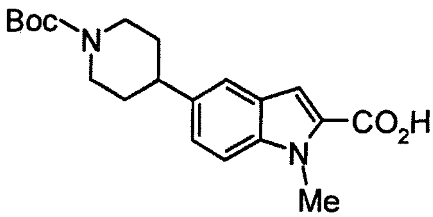 | ESI-: 357 |

[Table 10]

| Pre | Str | Dat |
|---|---|---|
| 18 | 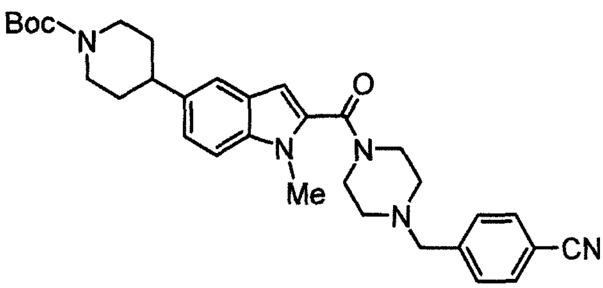 | ESI+: 542 |
| 19 | 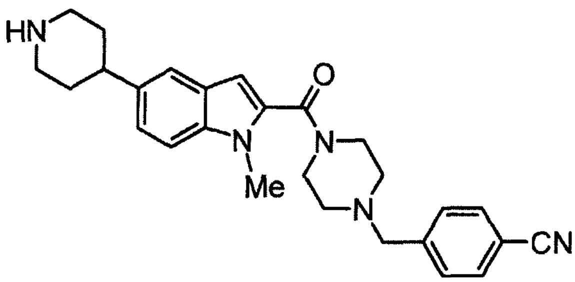 | ESI+: 442 |

[Table 11]

| Ex | Str |
|---|---|
| 1 | 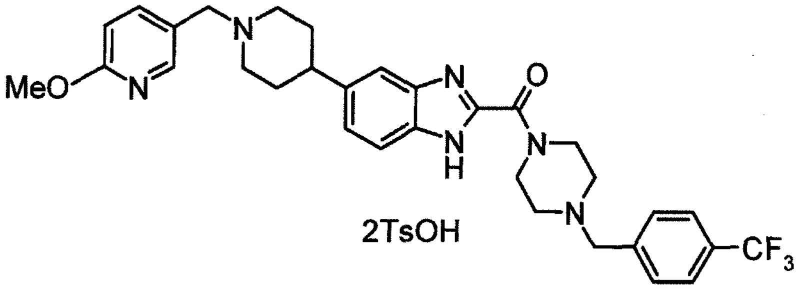 2TsOH |
| 2 | 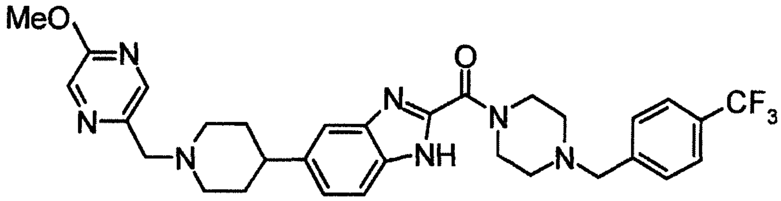 2TsOH |
| 3 | 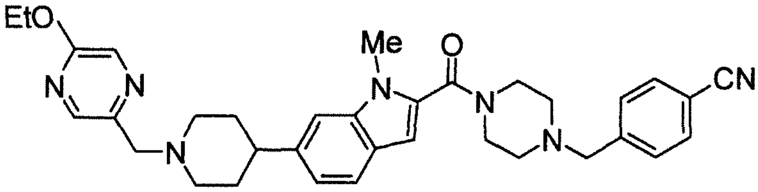 2TsOH |
| 4 | 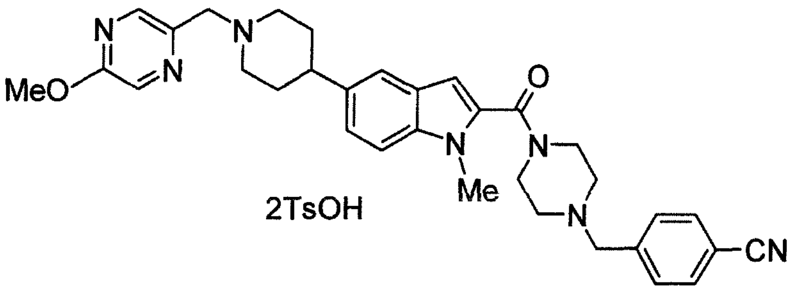 2TsOH |

[Table 12]

| Ex | Dat |
|---|---|
| 1 | ESI+: 593;<br>NMR1: 1.98-2.21 (4H, m), 2.38 (6H, s), 2.99-3.09 (1H, m), 3.12-3.27 (2H, m), 3.27-3.58 (8H, m), 3.59-3.68 (2H, m), 3.95 (3H, s), 4.36 (2H, s), 4.39-4.51 (2H, m), 6.86-6.92 (1H, m), 7.19-7.24 (4H, m), 7.25-7.30 (1H, m), 7.54-7.65 (2H, m), 7.68-7.83 (8H, m), 7.85 (1H, dd, J = 2.8, 8.4 Hz), 8.31 (1H, d, J = 2.0 Hz);<br>2θ(°) = 6.5, 10.1, 15.2, 16.2, 18.6, 19.6, 20.1, 20.8, 23.3, 25.8 |
| 2 | ESI+: 594;<br>NMR1: 2.01-2.22 (4H, m), 2.35 (6H, s), 3.01-3.11 (1H, m), 3.20-3.80 (12H, m), 4.02 (3H, s), 4.47 (2H, s), 4.52 (2H, s), 7.18-7.25 (4H, m), 7.31 (1H, dd, J = 1.6, 8.6 Hz), 7.55-7.59 (1H, m), 7.64 (1H, d, J = 8.5 Hz), 7.67-7.73 (4H, m), 7.75-7.78 (2H, m), 7.78-7.84 (2H, m), 8.31-8.35 (2H, m);<br>2θ(°) = 6.2, 6.7, 13.3, 15.2, 16.4, 19.0, 20.5, 20.9, 22.6, 24.8 |

(continued)

| Ex | Dat |
|---|---|
| 3 | ESI+: 578<br>NMR2: 1.38 (3H, t, J = 7.0 Hz), 1.89-2.10 (4H, m), 2.29 (6H, s), 2.88-3.00 (1H, m), 3.02-3.69 (12H, m), 3.76 (3H, s), 4.26-4.63 (4H, m), 4.41 (2H, q, J = 7.1 Hz), 6.61-6.79 (1H, m), 6.96-7.03 (1H, m), 7.06-7.14 (4H, m), 7.32 (1H, s), 7.44-7.51 (4H, m), 7.57 (1H, d, J = 8.3 Hz), 7.62-7.81 (2H, m), 7.85-8.10 (2H, m), 8.39 (1H, d, J = 1.2 Hz), 8.42 (1H, d, J = 1.2 Hz), 9.67-9.81 (1H, m), 9.89-10.16 (1H, m);<br>2θ(°) = 3.6, 7.2, 10.9, 16.1, 16.7, 17.2, 19.2, 20.9, 22.8, 26.6 |
| 4 | ESI+: 564<br>NMR2: 1.84-2.07 (4H, m), 2.29 (6H, s), 2.82-2.94 (1H, m), 3.08-3.68 (12H, m), 3.75 (3H, s), 3.97 (3H, s), 4.21-4.66 (4H, m), 6.57-6.80 (1H, m), 7.06-7.13 (4H, m), 7.12-7.19 (1H, m), 7.37-7.43 (1H, m), 7.44-7.54 (5H, m), 7.58-7.79 (2H, m), 7.84-8.10 (2H, m), 8.41 (1H, d, J = 1.2Hz), 8.46 (1H, d, J = 1.3Hz), 9.64-9.83 (1H, m), 9.87-10.16 (1H, m);<br>2θ(°) = 7.5, 9.8, 13.2, 14.7, 15.6, 16.9, 18.8, 19.5, 20.0, 22.6 |

Industrial Applicability

A compound selected from Compound A, Compound B, Compound C, and Compound D or a pharmaceutically acceptable salt thereof, which are active ingredients of a pharmaceutical composition of the present invention, has the effect of inhibiting mitochondrial Complex I, exhibits the effect of inhibiting growth of multiple myeloma, and can be used as an active ingredient of a pharmaceutical composition for treating multiple myeloma, and in another embodiment, of a pharmaceutical composition for treating multiple myeloma in which the PI3K/Akt/mTOR pathway is enhanced.

## Claims

**1.** A pharmaceutical composition for treating multiple myeloma, the composition comprising:

a compound selected from (5-{1-[(6-methoxypyridin-3-yl)methyl]piperidin-4-yl}-1H-benzimidazol-2-yl){4-[4-(trifluoromethyl)benzyl]piperazin-1-yl}methanone, (5-{1-[(5-methoxypyrazin-2-yl)methyl]piperidin-4-yl}-1H-benzimidazol-2-yl) {4-[4-(trifluoromethyl)benzyl]piperazin-1-yl)methanone, 4-({4-[(6-{1-[(5-ethoxypyrazin-2-yl)methyl]piperidin-4-yl}-1-methyl-1H-indol-2-yl)carbonyl]piperazin-1-yl}methyl)benzonitrile, and 4-({4-[(5-{1-[(5-methoxypyrazin-2-yl)methyl]piperidin-4-yl}-1-methyl-1H-indol-2-yl)carbonyl]piperazin-1-yl}methyl)benzonitrile, or a pharmaceutically acceptable salt thereof as an active ingredient.

**2.** The pharmaceutical composition according to claim 1, wherein the multiple myeloma is a multiple myeloma in which the PI3K/Akt/mTOR pathway is enhanced.

**3.** The pharmaceutical composition according to claim 2, comprising:

(5-{1-[(6-methoxypyridin-3-yl)methyl]piperidin-4-yl}-1H-benzimidazol-2-yl){4-[4-(trifluoromethyl)benzyl]piperazin-1-yl}methanone or a pharmaceutically acceptable salt thereof, and a pharmaceutically acceptable excipient.

**4.** The pharmaceutical composition according to claim 2, comprising:

(5-{1-[(5-methoxypyrazin-2-yl)methyl]piperidin-4-yl}-1H-benzimidazol-2-yl){4-[4-(trifluoromethyl)benzyl]piperazin-1-yl}methanone or a pharmaceutically acceptable salt thereof, and a pharmaceutically acceptable excipient.

**5.** The pharmaceutical composition according to claim 2, comprising:

4-({4-[(6-{1-[(5-ethoxypyrazin-2-yl)methyl]piperidin-4-yl}-1-methyl-1H-indol-2-yl)carbonyl]piperazin-1-yl}methyl)benzonitrile or a pharmaceutically acceptable salt thereof, and a pharmaceutically acceptable excipient.

**6.** The pharmaceutical composition according to claim 2, comprising:

4-({4-[(5-{1-[(5-methoxypyrazin-2-yl)methyl]piperidin-4-yl}-1-methyl-1H-indol-2-yl)carbonyl]piperazin-1-yl}methyl)benzonitrile or a pharmaceutically acceptable salt thereof, and a pharmaceutically acceptable excipient.

**7.** The pharmaceutical composition according to claim 2, comprising:

(5-{1-[(6-methoxypyridin-3-yl)methyl]piperidin-4-yl}-1H-benzimidazol-2-yl){4-[4-(trifluoromethyl)benzyl]piperazin-1-yl}methanone ditosylate, and a pharmaceutically acceptable excipient thereof.

**8.** The pharmaceutical composition according to claim 2, comprising:

(5-{1-[(5-methoxypyrazin-2-yl)methyl]piperidin-4-yl}-H-benzimidazol-2-yl){4-[4-(trifluoromethyl)benzyl]piperazin-1-yl}methanone ditosylate, and a pharmaceutically acceptable excipient thereof.

**9.** The pharmaceutical composition according to claim 2, comprising:

4-({4-[(6-{1-[(5-ethoxypyrazin-2-yl)methyl]piperidin-4-yl}-1-methyl-1H-indol-2-yl)carbonyl]piperazin-1-yl}methyl)benzonitrile ditosylate, and a pharmaceutically acceptable excipient thereof.

**10.** The pharmaceutical composition according to claim 2, comprising:

4-({4-[(5-{1-[(5-methoxypyrazin-2-yl)methyl]piperidin-4-yl}-1-methyl-1H-indol-2-yl)carbonyl]piperazin-1-yl}methyl)benzonitrile ditosylate, and a pharmaceutically acceptable excipient thereof.

**11.** The pharmaceutical composition according to any one of Claims 1 to 10, wherein lenalidomide is used in combination.

## INTERNATIONAL SEARCH REPORT

International application No.
PCT/JP2015/084341

A. CLASSIFICATION OF SUBJECT MATTER
*A61K31/496*(2006.01)i, *A61K31/454*(2006.01)i, *A61K31/497*(2006.01)i, *A61P35/00*(2006.01)i, *A61P43/00*(2006.01)i, *C07D401/14*(2006.01)n

According to International Patent Classification (IPC) or to both national classification and IPC

B. FIELDS SEARCHED

Minimum documentation searched (classification system followed by classification symbols)
A61K31/496, A61K31/454, A61K31/497, A61P35/00, A61P43/00, C07D401/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | WO 02/20008 A1 (THE SCRIPPS RESEARCH INSTITUTE), 14 March 2002 (14.03.2002), page 13, lines 8 to 23; page 14, lines 5 to 12; fig. 15A, 15B, 17 & US 2004/0034239 A1 & AU 8889901 A | 1-11 |
| P,A | WO 2014/199933 A1 (Astellas Pharma Inc.), 18 December 2014 (18.12.2014), claims 8 to 15; test examples 1 to 4; examples 1, 7, 66, 79 & US 2015/0266869 A1 & CA 2914982 A & TW 201536771 A | 1-11 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 March 2016 (04.03.16) | 15 March 2016 (15.03.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009132136 A
- WO 2012016217 A
- WO 2014199933 A


### Non-patent literature cited in the description


- *SEER Cancer Statistics Review,* 2008-2012
- *Leukemia,* 2014, vol. 28, 1122-1128
- *Clin. Cancer Res.,* 2007, vol. 13, 3771-3775
- *Oncogene,* 2001, vol. 20, 5991-6000
- *J.Immunol.,* 2004, vol. 173, 4953-4959
- *Clin. Cancer Res.,* 2011, vol. 17, 4693-4704
- *Cancer Res.,* 2006, vol. 66, 10269-10273
- *Expert Opin. Investig. Drugs.,* 2013, vol. 22, 1401-1409
- *Annu. Rev. Biochem.,* 1998, vol. 67, 821-855
- *Diabetes Metab.,* 2003, vol. 29 (4), 6S88-94
- *Genes Cells.,* 2003, vol. 8, 65-79
- *Cancer Res.,* 2007, vol. 67, 10804-10812